(19) Europäisches Patentamt
European Patent Office
Office européen des brevets

(11) Numéro de publication: **0 319 418 B1**

(12) **FASCICULE DE BREVET EUROPEEN**

(45) Date de publication de fascicule du brevet: **10.11.93**

(51) Int. Cl.$^5$: **C12N 15/00**, C12N 1/20, A01N 63/00

(21) Numéro de dépôt: **88403030.5**

(22) Date de dépôt: **01.12.88**

(54) **Nouveaux plasmides provoquant des densonucléoses, leur procédé d'obtention et leur application dans la lutte biologique contre les insectes ravageurs.**

(30) Priorité: **03.12.87 FR 8716764**

(43) Date de publication de la demande:
**07.06.89 Bulletin 89/23**

(45) Mention de la délivrance du brevet:
**10.11.93 Bulletin 93/45**

(84) Etats contractants désignés:
**AT BE CH DE GB IT LI NL SE**

(56) Documents cités:

**JOURNAL OF VIROLOGY, vol. 61, no. 2, février 1987, pages 553-560, American Society for Microbiology, US; H. BANDO et al.: "Organization and nucleotidesequence of a densovirus genome imply a host-dependent evolution of theparvoviruses"**

**ARCHIVES OF VIROLOGY, vol. 93, nos. 1-2, janvier 1987, pages 139-146, Springer-Verlag; H. Bando et al.**

(73) Titulaire: **ROUSSEL-UCLAF**
**35, Boulevard des Invalides**
**F-75007 Paris(FR)**

(72) Inventeur: **Dumas, Bruno**
**201, rue Lafayette**
**F-75010 Paris(FR)**
Inventeur: **Gervais, Monica**
**87, Chemin des Claies**
**F-95320 Saint Leu La Forêt(FR)**
Inventeur: **Bergoin, Max**
**111, rue des Acacias**
**F-30380 Saint Christophe Lez Ales(FR)**
Inventeur: **Jourdan, Mireille**
**Clairac Meyrannes**
**F-30410 Molière Sur Ceze(FR)**
Inventeur: **Jousset, Françoise Xavière**
**111, rue des Acacias**
**F-30380 Saint Christophe Lez Ales(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

COMPTE RENDUS DE L'ACADEMIE DES SCIENCES DE PARIS, vol. 299, no. 20, série III,1984, pages 889-894, Académie des Sciences, Paris, FR; F. ODIER: "PATHOLOGIE ANIMALE. - Etude comparée de la reconnaissance des infections à Densovirus parles méthodes cytologiques d'immunodiffusion et immunoenzymatique"

(74) Mandataire: **Bourgouin, André et al**
**Département des Brevets**
**ROUSSEL UCLAF**
**111, route de Noisy**
**B.P. no 9**
**F-93230 Romainville (FR)**

## Description

L'invention concerne de nouveaux plasmides provoquant des densonucléoses, leur procédé d'obtention et leur application dans la lutte biologique contre les insectes ravageurs.

Les densonucléoses sont les maladies affectant un grand nombre d'invertébrés, essentiellement des insectes, provoquées par les Densovirus. Le terme densonucléose reflète l'hypertrophie caractéristique des noyaux dans lesquels les Densovirus se multiplient. Dans des conditions naturelles, une mortalité avoisinant 100% peut être atteinte en 4 ou 5 jours.

Ces virus, classés dans le genre Densovirus, constituent avec les 2 genres Parvovirus et Dependovirus de parvovirus de vertébrés, la famille des Parvoviridae.

Les Densovirus sont de petits virus isométriques non enveloppés, à ADN, fortement pathogènes pour plusieurs espèces d'insectes ravageurs d'importance économique, notamment les lépidoptères chez lesquels ils ont été originellement découverts. Dans les milieux naturels, ils sont responsables d'épizooties qui participent efficacement à la régulation des populations de leurs hôtes. On a isolé à ce jour une vingtaine de Densovirus à partir d'insectes de différents groupes : lépidoptères, diptères, orthoptères, dictyoptères provenant de toutes les régions du globe.

Les densovirus présentent donc de très intéressantes potentialités d'utilisation vis-à-vis d'insectes d'importance économique et notamment de lépidoptères, ce nouveau moyen de lutte microbiologique pouvant se substituer à la lutte chimique ou la compléter.

Par ailleurs, il est important de souligner qu'en dépit de leur forte virulence vis-à-vis des insectes, des essais préliminaires et l'expérience de plusieurs années de laboratoire, n'ont pas révélé d' effets pathogènes pour l'homme.

Des préparations à base d'autres virus entomopathogènes comme les baculovirus sont commercialisées actuellement.

Les méthodes de production industrielle de ces virus, qui reposent sur l'infection d'insectes en élevage de masse, entrainent des coûts de production élevés. Les tentatives de production en masse de ces virus sur cultures cellulaires se sont heurtées aux mêmes problèmes de prix de revient.

Comme pour les baculovirus, la multiplication des densovirus est obtenue normalement sur des larves (Ann. Rev. Entomol. 1979 24 : 63 87), ce qui implique un coût élevé de production.

Par ailleurs, plusieurs souches de densovirus ne peuvent être multipliées sur leurs hôtes, compte tenu des grandes difficultés rencontrées pour mettre au point des milieux artificiels nécessaires aux élevages industriels.

Il était donc très intéressant de trouver d'autres voies de production du matériel infectieux, ne faisant pas intervenir la multiplication sur l'hôte. Or, la possibilité d'obtenir cette production par génie génétique est possible pour les densovirus. En effet, la taille de leur génome est compatible avec une insertion dans un plasmide bactérien alors qu'elle ne peut être envisagée pour un virus à génome de très haut poids moléculaire comme les baculovirus.

L'invention a donc pour objet de nouveaux plasmides recombinants capables de se répliquer dans Escherichia coli caractérisés en ce qu'ils contiennent une séquence complète ou partielle de l'ADN double ou simple brin d'un densovirus provoquant une densovirose chez un insecte sensible.

Tous les densovirus dont une séquence complète ou partielle de l'ADN est contenue dans les plasmides de l'invention sont cités dans Intervirology 23 : 61-73 (1985).

Les Densovirus de lépidoptères ravageurs les plus étudiés sont les Densovirus de Junonia, de Galleria et d' Agraulis.

L'invention concerne donc particulièrement des plasmides caractérisés en ce qu'ils contiennent une séquence complète ou partielle double ou simple brin d'un Densovirus de Junonia, de Galleria ou d'Agraulis et tout particulièrement des plasmides caractérisés en ce qu'ils contiennent une séquence complète ou partielle de l'ADN double ou simple brin du Densovirus de Junonia ( = J-DNV).Ces derniers plasmides sont appelés pJ ci-après dans le texte.

Le génome des densovirus est constitué d'une molécule d'ADN linéaire simple brin de 5 à 6000 bases. Au cours de la morphogénèse virale, les virions des densovirus encapsident indifféremment mais toujours séparément et en proportion équimoléculaire, des chaines + et des chaines -. L'extraction dans des conditions de force ionique élevée (O,I SSC) d'ADN à partir d'une telle population virale, conduit à la formation de molécules bicaténaires par appariement des chaines complémentaires. (Truffaut et al (1967) et Arch. Ges. Virusforsch. 21, 469-474. et Barvise, A. H., and walker, I.O. (1970). FEBS lett. 6, 13-16).
Ainsi, par leur faible poids moléculaire et leur propriété de former des chaines bicaténaires, ces génomes se prêtent particulièrement bien à leur intégration dans des plasmides. Il faut souligner que l'ADN bicaténaire extrait des virions est infectieux, aussi bien en cultures cellulaires que par transfection de larves de l'espèce sensible. (JOUSSET, 1er Congr. Soc. fr. Microbiol, Toulouse (France), Avril 1986, CP59, p. 44. et JOUSSET Soc. Invertebr. Pathol., 1986, p.121. Colloq. Invertebr., Veldhoven (The Netherlands), August 1986).

Le clonage de l'ADN bicaténaire du Densovirus de Junonia a été ainsi réalisé et est décrit plus loin dans la partie expérimentale.

La séquence du génome cloné ainsi obtenu a été déterminée selon les méthodes usuelles (Sanger et al (1977) Proc. Nat. Acad. Sci. USA 74 p. 5463 - Maxam A.M. et Gilbert W. (1980) Methods Enzymol. 65 p. 499).

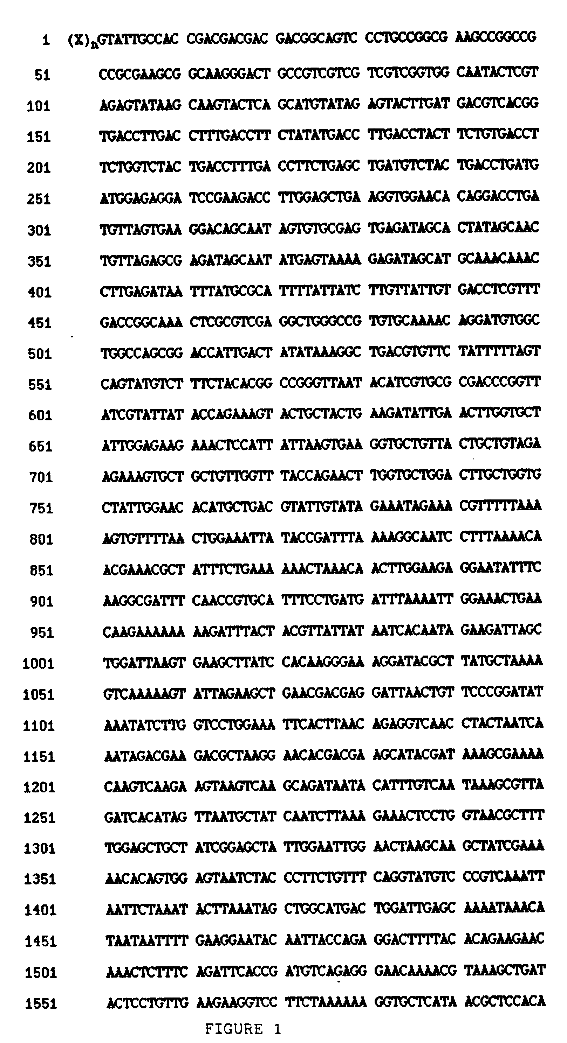

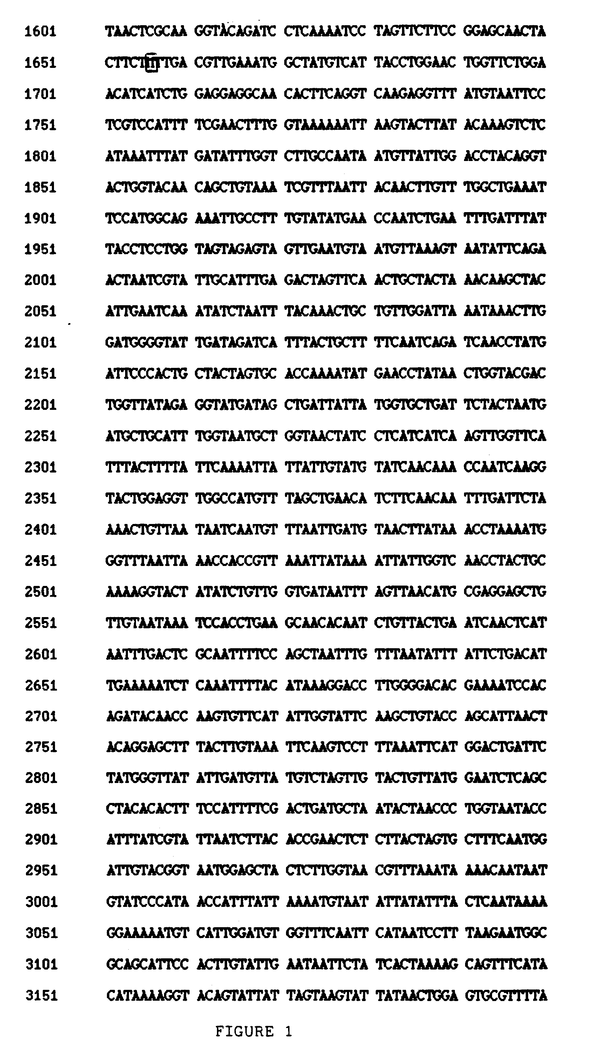

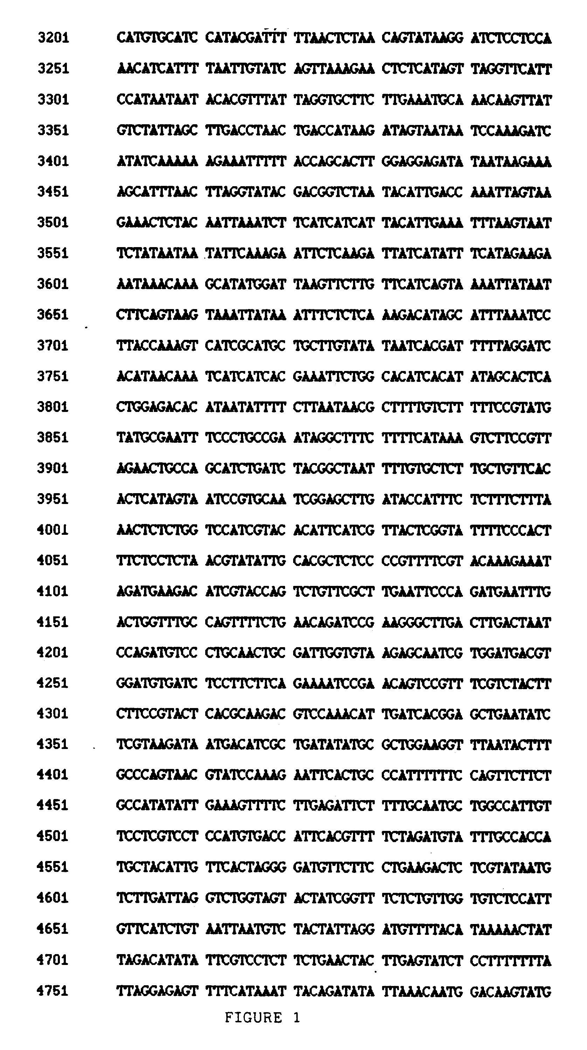

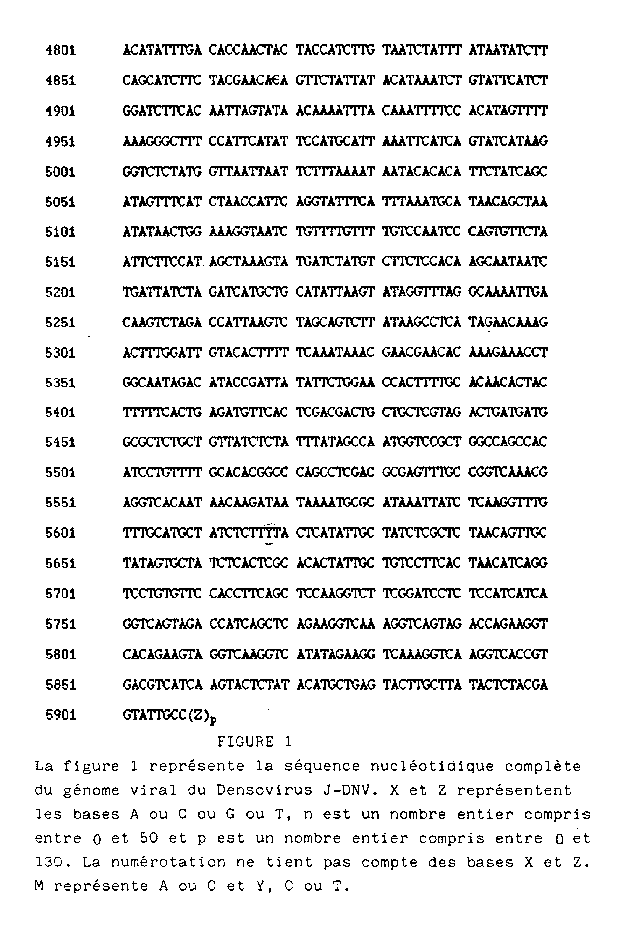

L'invention a donc aussi pour objet la séquence complète du génome viral du Densovirus J-DNV qui est donnée à la figure 1.

Elle concerne tout particulièrement la séquence complète de l'ADN viral du Densovirus J-DNV correspondant à la séquence donnée à la figure 1 dans laquelle sont éliminées les deux extrémités "X" et "Z".

L'invention concerne donc particulièrement les plasmides caractérisés en ce qu'ils contiennent la séquence complète du génome viral du Densovirus J-DNV donnée à la figure 1. L'invention a aussi particulièrement pour objet les plasmides caractérisés en ce qu'ils contiennent la séquence complète ou partielle de l'ADN double ou simple brin du Densovirus de Galleria. Ces plasmides sont appelés pG ci-après dans le texte. Parmi les vecteurs pJ définis ci-dessus, le vecteur pBRJ est le vecteur préféré.

Ce plasmide est caractérisé en ce qu'il renferme la séquence complète de l'ADN viral du Densovirus J-DNV cloné correspondant à la séquence donnée à la figure 1 dans laquelle sont éliminées les deux extrémités "X" et "Z" le principe de construction du plasmide est donné plus loin dans le texte et sa construction est détaillée dans la partie expérimentale.

La construction des plasmides selon l'invention fait appel aux techniques habituelles utilisées dans le domaine. Ces techniques sont celles décrites par exemple dans Maniatis Molecular cloning : A - Laboratory Manual, Cold Spring Harbor Laboratory (1982) ou dans Basics methods in molecul biology Davis - Dibner Battney - Elsevier (1986).

Le vecteur de départ utilisé dans la construction des plasmides selon l'invention est de préférence pBR 322 mais tout autre vecteur classique de clonage peut être utilisé, comme par exemple le vecteur $PUC_{18}$ - [Norrander, J et al, Gene 26, 101 - 106 (1983)] ou le vecteur $pAT_{153}$ [Twigg - A.J. and sheratt D, Nature 283,216 - 218 (1980)].

Ce vecteur de départ est digéré par une ou plusieurs enzymes de restriction, dans un site de clônage qui est de préférence un site unique.

Le choix de ce site est dicté par les connaissances antérieures concernant la cartographie du génome viral du Densovirus. Ainsi, les plasmides pJ déjà définis précédemment, peuvent être construits à partir du vecteur de départ pBR 322 digéré par des enzymes générant des bouts francs comme par exemple ECoR V ou NrUI ou des enzymes générant des bouts collants comme par exemple Pst I.

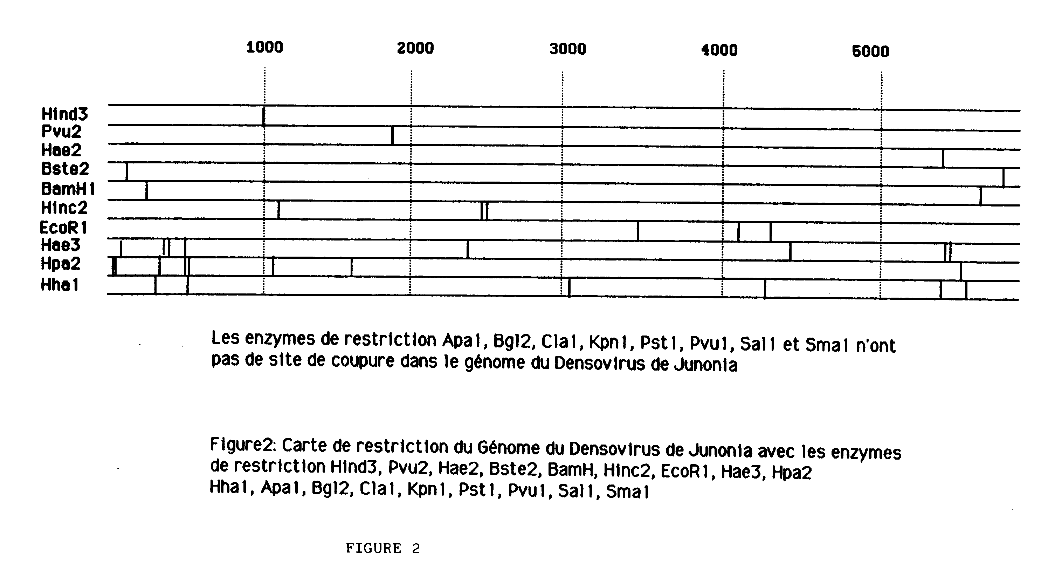

Ces différentes possibilités découlent de la cartographie du génome viral J-DNV donnée à la figure 2.

La même stratégie de clonage peut bien entendu s'appliquer aux autres plasmides de l'invention, comme par exemple aux plasmides pG déjà définis précédemment qui sont contruits en utilisant l'enzyme de restriction PstI.

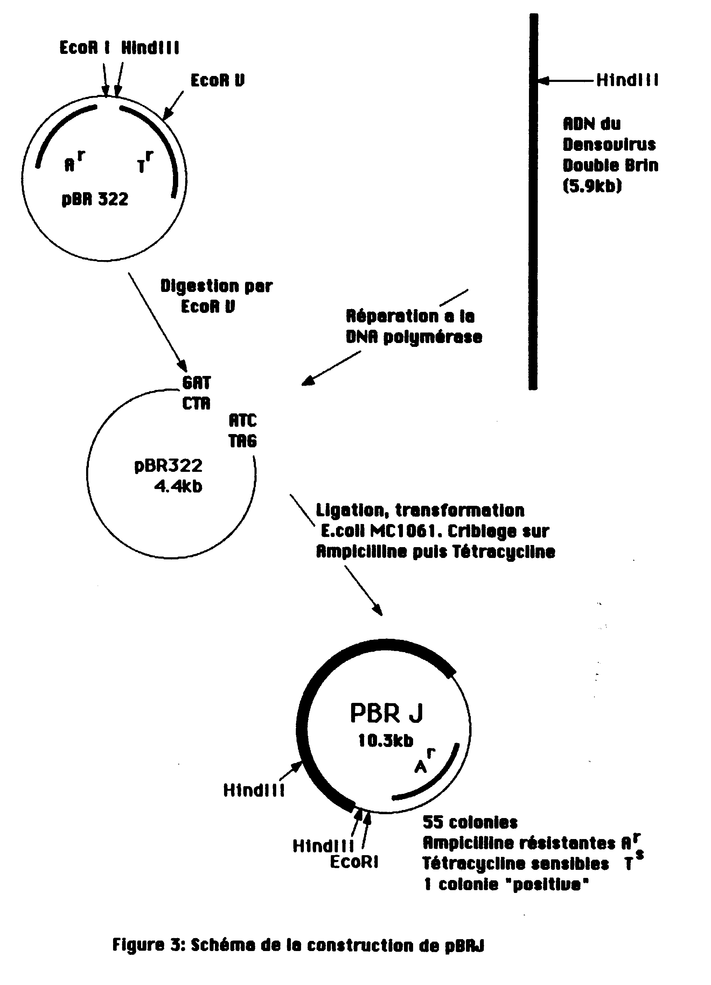

La construction du plasmide préféré de l'invention, le plasmide pBRJ, est détaillé à la figure 3. L'ADN viral a été obtenu à partir du virus Junonia selon les techniques connues d'extraction et de purification. L'ADN purifié, après traitement à la polymérase est intégré au site unique ECoR V de pBR 322 linéarisé.

Après transformation dans Escherichia coli, seules les colonies résistantes à l'ampicilline et sensibles à la tétracycline sont selectionnées.

Des mini-lyses des bactéries recombinantes sont effectuées afin de ne retenir que les colonies renfermant les plasmides ayant inséré un fragment de taille voisine à celle de l'ADN génomique.

Le schéma de construction de pBRJ est donné à la figure 3.

L'invention concerne par ailleurs les bactéries hôtes Escherichia coli transformées par les plasmides recombinants capables de se répliquer dans Escherichia coli caractérisés en ce qu'ils contiennent une séquence complète ou partielle de l'ADN double ou simple brin d'un densovirus provoquant une densovirose chez un insecte sensible. Elle concerne notamment les bactéries tranformées par les plasmides pJ et tout particulièrement par le plasmide pBRJ.

Tous les plasmides tels que définis ci-dessus et notamment les plasmides pJ et parmi ceux-ci le plasmide pBRJ, peuvent être utilisés pour lutter contre les insectes ravageurs.

Les essais décrits dans la partie expérimentale montrent en effet que la transfection par injection du plasmide pBRJ dans l'hémolymphe de larves de Spodoptera permet de reproduire une densovirose identique à celle provoquée par inoculation du virion ou de l'ADN viral J.

La même sensibilité au pBRJ a été démontrée sur des souches de Spodoptera résistantes aux insecticides chimiques comme la deltamétrine.

L'invention concerne donc l'application dans la lutte biologique contre les insectes ravageurs, des plasmides de l'invention et notamment des plasmides pJ et parmi ceux-ci, l'application du plasmide pBRJ.

Les insectes d'importance économique concernés par une telle application sont certains diptères vecteurs, d'intérêt médical et vétérinaire et des lépidoptères et coléoptères ravageurs de cultures vivrières et industrielles (coton, maïs, soja, palmier à huile, bananier).

L'invention englobe également toutes les formulations insecticides adaptées à une telle application.

Les formulations adéquates sont celles qui permettent un enrobage de l'ADN des plasmides de l'invention. Les vésicules enrobant cet ADN doivent répondre à plusieurs critères :

- cavité interne hydrophile, de diamètre suffisant.
- assurer la protection de l'ADN du plasmide dans le tractus intestinal.
- libérer leur contenu au niveau des cellules cibles, celles de l'intestin moyen par exemple.

De telles vésicules sont par exemple des niosomes, des liposomes, des microcapsules de gélatine ou de polymères hydrosolubles ou d'acrylamide ou tout autre type de microcapsules compatibles avec les critères définis ci-dessus. Des formes d'encapsidation dans des phages monocaténaires du type M 13 sont aussi à envisager.

Les résultats expérimentaux donnés ci-dessous illustrent l'invention sans toutefois la limiter.

<u>Construction du plasmide pBRJ</u>

1) Purification de l'ADN du virus
2) Clonage de l'ADN du virus
3) Séquence de l'ADN du virus
4) Résultats des transfections des larves de Spodoptera littoralis par l'ADN du Densovirus cloné ou non dans pBR 322.

<u>1) Purification de l'ADN</u>

a) <u>Purification du virus.</u>

20 à 30 larves Spodoptera littoralis infectées par le Densovirus de Junonia sont broyées au Potter dans du tampon TrisHCl 0,1M pH 7,4 acide ascorbique 2%. Le broyat est clarifié par une centrifugation de 10 minutes à 10.000 g. Les virions sont ensuite culottés par une centrifugation de 90 minutes à 35000 rpm dans un rotor SW41 à 4°C. Le culot est repris et déposé sur un gradient de rénographine 20-76% et centrifugé 15 heures à 35000 rpm dans un rotor SW41. La bande de virus localisée dans le 1/3 inférieur du gradient est récupérée puis dialysée contre du tampon TE (Tris HCl 10mM EDTA 1mM pH 8.0) pendant 3 jours avec un changement de tampon toutes les 12 heures. La pureté des suspensions virales est ensuite contrôlée par microscopie électronique et spectrophotométrie UV. Leur concentration est estimée par mesure de la densité optique à 260 nm. Une unité de DO à 260 nm est équivalente à 100µg de virion par ml.

<u>b) Extraction de l'ADN</u>

Le tampon de la suspension virale est ajusté à 4mM EDTA, à 100ug de protéinase K par ml et à 2% sarkosyl.

Après une incubation de 2 heures à 37°C, la suspension est extraite trois fois par un même volume de phénol saturé de tampon TE. La phase aqueuse est récupérée puis dialysée contre du tampon TE. L'ADN est ensuite précipité par addition d'acétate de sodium ou de chlorure de lithium à 0.2M final et deux volumes d'éthanol de 12 heures à 16 heures à -20°C.

Le culot d'ADN est rincé trois fois à l'alcool à 70% puis repris dans du tampon TE. La pureté de la solution d'ADN viral est contrôlée et sa concentration déterminée par spectrophotométrie UV.

2) Clonage de l'ADN du virus.

a) Préparation de l'ADN du virus.

Les extrémités du virus sont réparées par l'ADN polymérase de E. Coli fragment de Klenow.

3µg d'ADN du virus sont réparés dans un volume de 75µl à l'aide de 7,5U d'ADN polymérase de E.Coli (Boehringer Mannheim Biochemicals = BMB cat n° 1014531). Le tampon de réparation contient les deoxyadénosine, deoxycytosine, guanosine et thymidine à la concentration de 40µM, du $MgCl_2$ 5mM, bétamercaptoéthanol 10mM, de la sérum albumine de boeuf 100µg/ml et du Tris HCl 10mM pH 7,5. La réaction est incubée à 25°C pendant une heure. Après une heure à 65°C, la réaction est extraite au phénol saturé Tris pH 7,5 0,1M puis précipitée en présence d'un volume d'acétate 0,3M et deux volumes d'éthanol 100%.

b) Préparation de l'ADN du vecteur pBR322

L'ADN de PBR322 est préparé selon la méthode décrite par Maniatis et Coll. Molecular Cloning : A. Laboratory Manual, Cold Spring Harbor Laboratory (1982). 2,5ug d'ADN de pBR322 sont digérés par l'enzyme de restriction EcoRV dans les conditions décrites par le fournisseur BMB en utilisant 18U/µg d'ADN d'enzyme dans un volume de 50µl pendant 4 heures à 37°C. La digestion de l'ADN est vérifiée sur un gel d'agarose à 1%. L'ADN est ensuite déphosphorylé à l'aide de la phosphatase alcaline d'intestin de veau. On ajoute donc 2µl de phosphatase alcaline BMB cat n° 713023 à 22U/µl. Après une incubation d'une heure à 37°C, la réaction est arrêtée par une incubation de 45 minutes à 65°C. Après une extraction au phénol saturé Tris 0,1M pH 7,5. La phase aqueuse est précipitée à l'éthanol en présence d'acétate 0,3M.

c) Ligation de l'ADN du Virus Junonia sur le vecteur pBR322 linéarisé par l'enzyme de restriction EcoRV.

Après précipitation et séchage, l'ADN du vecteur est repris à une concentration de 40ng/µl, l'ADN du virus est repris à une concentration de 1µg/µl. La ligation est pratiquée dans un volume de 25µl dans les conditions décrites par le fabricant (New England Biolabs). Après une incubation d'une nuit à 4°C. Le mélange est prêt pour transformer les bactéries Escherichia Coli (MC1061). (Casabadan MJ. Methods in Enzymol. (1983) 100 p.293-3080).

d) transformation des bactéries compétentes.

Les bactéries compétentes sont préparées selon le protocole décrit par Hanahan (Maniatis 1982) et conservées à -70°C sous forme de fractions de 200µl. A 100µl de bactéries compétentes décongelées à 37°C, sans dépassér 0°C, on ajoute 1µl de la solution précédente contenant l'ADN du virus et le vecteur pBR322. Après une incubation de 15 minutes à 0°C puis un choc thermique de 5 à 37°C, on ajoute 900µl de milieu LB (10g Bactotryptone DIFCO, 5g Yeast extract DIFCO, 5g NaCl par litre Autoclaver à 120°C). Les bactéries recombinantes sont sélectionnées sur des boîtes de milieu LB gélosé contenant de l'Ampicilline à 100µg/ml. Sur 253 colonies résistantes à l'Ampicilline 50 colonies sensibles à la tétracycline sont sélectionnées.

e) mini-lyse des bactéries recombinantes et obtention du pBRJ.

Parmi ces bactéries recombinantes sensibles à la tétracycline une seule porte l'ADN du virus cloné au site EcoRV. A partir d'une culture (1,5ml) en milieu L-B de chacun des clones recombinants, les ADN plasmidiques ont été préparés selon la méthode alcaline (Maniatis 1982). Chacun des ADN a été digéré par l'enzyme de restriction EcoR1. Une seule colonie porte un ADN plasmidique correspondant à un ADN de pBR322 avec du Densovirus de Junonia inséré au site EcoRV détruit lors du clonage. Le plasmide correspondant pBRJ est préparé en grande quantité à partir d'un litre de culture à saturation en milieu L-B par la méthode à l'hydroxyde de sodium et au sodium dodécyl sulfate (Maniatis 1982). Des digestions par les enzymes de Restriction BamH1, Pvu2, Bgl2, Nru1, Acc1, Pvu1, BstE2, Sph1, EcoR1, Hind3, Rsa1, Bgl1 ont permis de vérifier que l'ADN du Densovirus était inséré au site EcoRV détruit pendant le clonage dans l'orientation décrite sur la figure 3.

3) Séquence de l'ADN du Densovirus de Junonia.

La séquence de l'ADN du virus a été déterminée par la méthode de Sanger (Sanger 1977) pour sa plus grande partie. La jonction virus pBR322 du côté du site Hind 3 de pBR322 est séquencée par la méthode de Maxam et Gilbert.

a) méthode de Sanger.

1) Clonage au hasard.
méthode enzymatique.

Des clonages au hasard sont réalisés avec les enzymes de restriction :
Sau3A1, Xho2, Alu1, Hae3, Acc1. L'ADN du virus a été digéré par ces enzymes de restriction qui coupent fréquemment l'ADN du Densovirus de Junonia pour obtenir des fragments de l'ordre de 100 paires de bases (pb) à 1000 pb. Les fragments obtenus sont sous-clonés dans les vecteurs M13mp8 et M13mp9 (Messing, J. et Viera, Gene, 19, 259-268 (1982)) digérés par l'enzyme BamH1 (digestions Sau3A, Xho2), par l'enzyme Sma1 (digestions Alu1, Hae3) et par l'enzyme Acc1 (digestion Acc1).

- méthode par sonication.

l'ADN du virus a été soniqué jusqu'à obtenir des fragments de l'ordre de 500pb. Les fragments obtenus ont été réparés à l'aide de la DNA polymérase de E.Coli fragment de Klenow selon le protocole décrit précédemment. Les fragments obtenus sont ensuite sous clonés dans les vecteurs M13mp8 et M13mp9 digérés par l'enzyme Sma1.

2) Sous clonage de fragments identifiés d'après la cartographie de restriction.

On réalise le sous-clonage des fragments de restrictions identifiés dont la taille est inférieure à 2000pb, obtenus par digestions avec les enzymes de restrictions (voir cartographie de restriction à la figure 2). BamH1, EcoR1, Hind3-BstE2, BstE2-BamH1, BstE2-Hind3, Hind3-Pvu2, Xba1-BstE2, Xba1, Hae3, Pvu2-Hae3, EcoR1-Xba1, Xba1-BstE2, Hae3-EcoR1, Hae3-Xba1, EcoR1-Pvu2, Hae3-BstE2. Les fragments d'un poids moléculaire de 110pb à 2000pb obtenus par digestion avec les enzymes de restriction ci-dessus sont sous clonés puis séquencés de proche en proche en utilisant des amorces (Sanger 1977) Proc.Nat.Acad.Sci. USA 74 p.5463 ; Biggin (1983) Proc.Nat.Acad.Sci. 80 p. 3963).

b) Méthode de Maxam et Gilbert.

Un fragment de 375 pb Hind3 et BsteII, proche du site EcoR1 de pBR322 s'est révélé impossible à séquencer en utilisant la méthode de Sanger. La séquence de ce fragment a été obtenue à partir du site Hind3 et du site BstE2 en marquant avec la T4 DNA polynucléotide kinase et l'ATP 32p ou avec la DNA polymérase fragment de Klenow et le dTTP $^{32}$ p ou dCTPP$^{32}$ (Maxam et Gilbert). Les réactions sont mises à migrer sur un gel dénaturant acrylamide, urée (Sanger and Carbon (1978) F.E.B.S. lett. 87, p. 107) en présence de formamide à 40%.

c) Organisation des séquences.

Les séquences obtenues sont rentrées dans un ordinateur de type Microvax2. Elles sont comparées à l'aide des programmes du DB SYSTEM écrit par R.STADEN. (Nucleic Acids Research 12 (1) Juin 1987, p.387-395).
Après édition des séquences à l'aide du programme d'édition EDIT sous VAX/VMS, les séquences sont comparées entre elles à l'aide du programme DBCOMP de STADEN puis sont organisées à l'aide du programme DBUTIL. La séquence consensus obtenue est présentée à la figure 1.

4) Résultats des transfections des larves de spodoptera littoralis par l'ADN du Densovirus cloné ou non dans pBR322.

a) Protocole de transfection par inoculation.

Différentes concentrations d'ADN J ou de pBRJ sont inoculées à des jeunes larves de Spodoptera littoralis (stade 2 ou 3) dans un tampon TE, pH 8,0 (Tris 10mM, EDTA 1mM) additionné de DEAE-dextran (2 mg/ml final).

b) Surveillance des larves.

Les larves transfectées et celles des lots témoins sont élevées individuellement sur milieu axénique à 25°C.

Chaque jour

- les larves mortes sont prélevées,
- la date de nymphose est notée,
- les nymphes sont prélevées 6 jours après la nymphose.

c) Contrôle des insectes transfectés.

Chaque individu est broyé individuellement dans 1ml de PBS +2% azoture de sodium. Les broyats sont clarifiés et la présence de virus recherchée dans les surnageants par la méthode indirecte ELISA, en utilisant des anticorps titrés anti Densovirus Junonia préparés sur souris et des antiIgG de souris marquées à la peroxidase. Certains échantillons sont contrôlés au microscope électronique.

d) Résultats.

La transfection des jeunes larves de Spodoptera littoralis par inoculation d'ADNJ ou d'ADN de pBRJ aux concentrations de 30ng d'ADN-J par larve et de 56ng pBRJ/larve a conduit à un pourcentage d'infection supérieur à 80% dans les deux cas. Des résultats comparables sont obtenus en transfectant des larves de Spodoptera littoralis d'origine malgache résistante à la deltaméthrine.

Par ailleurs, aucune infection n'est apparue dans les lots témoins inoculés soit avec du tampon d'extraction TE soit avec de l'ADN pBR322 à la concentration de 30ng à 1ng/larve.

**Exemple de composition insecticide.**

| | | |
|---|---|---|
| Liposome à base de | { phosphatidyl choline............ | 5 mg |
| | { phosphatidyl sérine............. | 5 mg |
| | { cholestérol..................... | 2,5mg |
| pBRJ............................................ | | 28 µg |
| Eau q.s.p....................................... | | 1 ml. |

## Revendications

**1.** Séquence complète du génome viral du Densovirus de Junonia (J-DNV).

```
  1   (X)nGTATTGCCAC CGACGACGAC GACGGCAGTC CCTGCCGGCG AAGCCGGCCG
 51       CCGCGAAGCG GCAAGGGACT GCCGTCGTCG TCGTCGGTGG CAATACTCGT
101       AGAGTATAAG CAAGTACTCA GCATGTATAG AGTACTTGAT GACGTCACGG
151       TGACCTTGAC CTTTGACCTT CTATATGACC TTGACCTACT TCTGTGACCT
201       TCTGGTCTAC TGACCTTTGA CCTTCTGAGC TGATGTCTAC TGACCTGATG
251       ATGGAGAGGA TCCGAAGACC TTGGAGCTGA AGGTGGAACA CAGGACCTGA
301       TGTTAGTGAA GGACAGCAAT AGTGTGCGAG TGAGATAGCA CTATAGCAAC
351       TGTTAGAGCG AGATAGCAAT ATGAGTAAAA GAGATAGCAT GCAAACAAAC
401       CTTGAGATAA TTTATGCGCA TTTTATTATC TTGTTATTGT GACCTCGTTT
451       GACCGGCAAA CTCGCGTCGA GGCTGGGCCG TGTGCAAAAC AGGATGTGGC
501       TGGCCAGCGG ACCATTGACT ATATAAAGGC TGACGTGTTC TATTTTTAGT
551       CAGTATGTCT TTCTACACGG CCGGGTTAAT ACATCGTGCG CGACCCGGTT
601       ATCGTATTAT ACCAGAAAGT ACTGCTACTG AAGATATTGA ACTTGGTGCT
651       ATTGGAGAAG AAACTCCATT ATTAAGTGAA GGTGCTGTTA CTGCTGTAGA
701       AGAAAGTGCT GCTGTTGGTT TACCAGAACT TGGTGCTGGA CTTGCTGGTG
751       CTATTGGAAC ACATGCTGAC GTATTGTATA GAAATAGAAA CGTTTTTAAA
801       AGTGTTTTAA CTGGAAATTA TACCGATTTA AAAGGCAATC CTTTAAAACA
851       ACGAAACGCT ATTTCTGAAA AAACTAAACA ACTTGGAAGA GGAATATTTC
```

901 AAGGCGATTT CAACCGTGCA TTTCCTGATG ATTTAAAATT CGAAACTGAA

951 CAAGAAAAAA AAGATTTACT ACGTTATTAT AATCACAATA GAAGATTAGC

1001 TGGATTAAGT GAAGCTTATC CACAAGGGAA AGGATACGCT TATGCTAAAA

1051 GTCAAAAAGT ATTAGAAGCT GAACGACGAG GATTAACTGT TCCCGGATAT

1101 AAATATCTTG GTCCTGGAAA TTCACTTAAC AGAGGTCAAC CTACTAATCA

1151 AATAGACGAA GACGCTAAGG AACACGACGA AGCATACGAT AAAGCGAAAA

1201 CAAGTCAAGA AGTAAGTCAA GCAGATAATA CATTTGTCAA TAAAGCGTTA

1251 GATCACATAG TTAATGCTAT CAATCTTAAA GAAACTCCTG GTAACGCTTT

1301 TGGAGCTGCT ATCGGAGCTA TTGGAATTGG AACTAAGCAA GCTATCGAAA

1351 AACACAGTGG AGTAATCTAC CCTTCTGTTT CAGGTATGTC CCGTCAAATT

1401 AATTCTAAAT ACTTAAATAG CTGGCATGAC TGGATTGAGC AAAATAAACA

1451 TAATAATTTT GAAGGAATAC AATTACCAGA GGACTTTTAC ACAGAAGAAC

1501 AAACTCTTTC AGATTCACCG ATGTCAGAGG GAACAAAACG TAAAGCTGAT

1551 ACTCCTGTTG AAGAAGGTCC TTCTAAAAAA GGTGCTCATA ACGCTCCACA

1601 TAACTCGCAA GGTACAGATC CTCAAAATCC TAGTTCTTCC GGAGCAACTA

1651 CTTCTTTTGA CGTTGAAATG GCTATGTCAT TACCTGGAAC TGGTTCTGGA

1701 ACATCATCTG GAGGAGGCAA CACTTCAGGT CAAGAGGTTT ATGTAATTCC

1751 TCGTCCATTT TCGAACTTTG GTAAAAAATT AAGTACTTAT ACAAAGTCTC

1801 ATAAATTTAT GATATTTGGT CTTGCCAATA ATGTTATTGG ACCTACAGGT

1851 ACTGGTACAA CAGCTGTAAA TCGTTTAATT ACAACTTGTT TGGCTGAAAT

1901 TCCATGGCAG AAATTGCCTT TGTATATGAA CCAATCTGAA TTTGATTTAT

1951 TACCTCCTGG TAGTAGAGTA GTTGAATGTA ATGTTAAAGT AATATTCAGA

2001 ACTAATCGTA TTGCATTTGA GACTAGTTCA ACTGCTACTA AACAAGCTAC

2051 ATTGAATCAA ATATCTAATT TACAAACTGC TGTTGGATTA AATAAACTTG

2101 GATGGGGTAT TGATAGATCA TTTACTGCTT TTCAATCAGA TCAACCTATG

2151 ATTCCCACTG CTACTAGTGC ACCAAAATAT GAACCTATAA CTGGTACGAC

2201 TGGTTATAGA GGTATGATAG CTGATTATTA TGGTGCTGAT TCTACTAATG

2251 ATGCTGCATT TGGTAATGCT GGTAACTATC CTCATCATCA AGTTGGTTCA

2301 TTTACTTTTA TTCAAAATTA TTATTGTATG TATCAACAAA CCAATCAAGG

2351 TACTGGAGGT TGGCCATGTT TAGCTGAACA TCTTCAACAA TTTGATTCTA

2401 AAACTGTTAA TAATCAATGT TTAATTGATG TAACTTATAA ACCTAAAATG
2451 GGTTTAATTA AACCACCGTT AAATTATAAA ATTATTGGTC AACCTACTGC
2501 AAAAGGTACT ATATCTGTTG GTGATAATTT AGTTAACATG CGAGGAGCTG
2551 TTGTAATAAA TCCACCTGAA GCAACACAAT CTGTTACTGA ATCAACTCAT
2601 AATTTGACTC GCAATTTTCC AGCTAATTTG TTTAATATTT ATTCTGACAT
2651 TGAAAAATCT CAAATTTTAC ATAAAGGACC TTGGGGACAC GAAAATCCAC
2701 AGATACAACC AAGTGTTCAT ATTGGTATTC AAGCTGTACC AGCATTAACT
2751 ACAGGAGCTT TACTTGTAAA TTCAAGTCCT TTAAATTCAT GGACTGATTC
2801 TATGGGTTAT ATTGATGTTA TGTCTAGTTG TACTGTTATG GAATCTCAGC
2851 CTACACACTT TCCATTTTCG ACTGATGCTA ATACTAACCC TGGTAATACC
2901 ATTTATCGTA TTAATCTTAC ACCGAACTCT CTTACTAGTG CTTTCAATGG
2951 ATTGTACGGT AATGGAGCTA CTCTTGGTAA CGTTTAAATA AAACAATAAT
3001 GTATCCCATA ACCATTTATT AAAATGTAAT ATTATATTTA CTCAATAAAA
3051 GGAAAAATGT CATTGGATGT GGTTTCAATT CATAATCCTT TAAGAATGGC
3101 GCAGCATTCC ACTTGTATTG AATAATTCTA TCACTAAAAG CAGTTTCATA
3151 CATAAAAGGT ACAGTATTAT TAGTAAGTAT TATAACTGGA GTGCGTTTTA
3201 CATGTGCATC CATACGATTT TTAACTCTAA CAGTATAAGG ATCTCCTCCA
3251 AACATCATTT TAATTGTATC AGTTAAAGAA CTCTCATAGT TAGGTTCATT
3301 CCATAATAAT ACACGTTTAT TAGGTGCTTC TTGAAATGCA AACAAGTTAT
3351 GTCTATTAGC TTGACCTAAC TGACCATAAG ATAGTAATAA TCCAAAGATC
3401 ATATCAAAAA AGAAATTTTT ACCAGCACTT GGAGGAGATA TAATAAGAAA
3451 AGCATTTAAC TTAGGTATAC GACGGTCTAA TACATTGACC AAATTAGTAA
3501 GAAACTCTAC AATTAAATCT TCATCATCAT TACATTGAAA TTTAAGTAAT
3551 TCTATAATAA TATTCAAAGA ATTCTCAAGA TTATCATATT TCATAGAAGA
3601 AATAAACAAA GCATATGGAT TAAGTTCTTG TTCATCAGTA AAATTATAAT
3651 CTTCAGTAAG TAAATTATAA ATTTCTCTCA AAGACATAGC ATTTAAATCC
3701 TTACCAAAGT CATCGCATGC TGCTTGTATA TAATCACGAT TTTTAGGATC
3751 ACATAACAAA TCATCATCAC GAAATTCTGG CACATCACAT ATAGCACTCA
3801 CTGGAGACAC ATAATATTTT CTTAATAACG CTTTTGTCTT TTTCCGTATG
3851 TATGCGAATT TCCCTGCCGA ATAGGCTTTC TTTTCATAAA GTCTTCCGTT

```
3901    AGAACTGCCA GCATCTGATC TACGGCTAAT TTTGTGCTCT TGCTGTTCAC
3951    ACTCATAGTA ATCCGTGCAA TCGGAGCTTG ATACCATTTC TCTTTCTTTA
4001    AACTCTCTGG TCCATCGTAC ACATTCATCG TTACTCGGTA TTTTCCCACT
4051    TTCTCCTCTA ACGTATATTG CACGCTCTCC CCGTTTTCGT ACAAAGAAAT
4101    AGATGAAGAC ATCGTACCAG TCTGTTCGCT TGAATTCCCA GATGAATTTG
4151    ACTGGTTTGC CAGTTTTCTG AACAGATCCG AAGGGCTTGA CTTGACTAAT
4201    CCAGATGTCC CTGCAACTGC GATTGGTGTA AGAGCAATCG TGGATGACGT
4251    GGATGTGATC TCCTTCTTCA GAAAATCCGA ACAGTCCGTT TCGTCTACTT
4301    CTTCCGTACT CACGCAAGAC GTCCAAACAT TGATCACGGA GCTGAATATC
4351    TCGTAAGATA ATGACATCGC TGATATATGC GCTGGAAGGT TTAATACTTT
4401    GCCCAGTAAC GTATCCAAAG AATTCACTGC CCATTTTTTC CAGTTCTTCT
4451    GCCATATATT GAAAGTTTTC TTGAGATTCT TTTGCAATGC TGGCCATTGT
4501    TCCTCGTCCT CCATGTGACC ATTCACGTTT TCTAGATGTA TTTGCCACCA
4551    TGCTACATTG TTCACTAGGG GATGTTCTTC CTGAAGACTC TCGTATAATG
4601    TCTTGATTAG GTCTGGTAGT ACTATCGGTT TCTCTGTTGG TGTCTCCATT
4651    GTTCATCTGT AATTAATGTC TACTATTAGG ATGTTTTACA TAAAAACTAT
4701    TAGACATATA TTCGTCCTCT TCTGAACTAC TTGAGTATCT CCTTTTTTTA
4751    TTAGGAGAGT TTTCATAAAT TACAGATATA TTAAACAATG GACAAGTATG
4801    ACATATTTGA CACCAACTAC TACCATCTTG TAATCTATTT ATAATATCTT
4851    CAGCATCTTC TACGAACACA GTTCTATTAT ACATAAATCT GTATTCATCT
4901    GGATCTTCAC AATTAGTATA ACAAAATTTA CAAATTTTCC ACATAGTTTT
4951    AAAGGGCTTT CCATTCATAT TCCATGCATT AAATTCATCA GTATCATAAG
5001    GGTCTCTATG GTTAATTAAT TCTTTAAAAT AATACACACA TTCTATCAGC
5051    ATAGTTTCAT CTAACCATTC AGGTATTTCA TTTAAATGCA TAACAGCTAA
5101    ATATAACTGG AAAGGTAATC TGTTTTGTTT TGTCCAATCC CAGTGTTCTA
5151    ATTCTTCCAT AGCTAAAGTA TGATCTATGT CTTCTCCACA AGCAATAATC
5201    TGATTATCTA GATCATGCTG CATATTAAGT ATAGGTTTAG GCAAAATTGA
5251    CAAGTCTAGA CCATTAAGTC TAGCAGTCTT ATAAGCCTCA TAGAACAAAG
5301    ACTTTGGATT GTACACTTTT TCAAATAAAC GAACGAACAC AAAGAAACCT
5351    GGCAATAGAC ATACCGATTA TATTCTGGAA CCACTTTTGC ACAACACTAC
```

```
5401   TTTTTCACTG AGATGTTCAC TCGACGACTG CTGCTCGTAG ACTGATGATG
5451   GCGCTCTGCT GTTATCTCTA TTTATAGCCA ATGGTCCGCT GGCCAGCCAC
5501   ATCCTGTTTT GCACACGGCC CAGCCTCGAC GCGAGTTTGC CGGTCAAACG
5551   AGGTCACAAT AACAAGATAA TAAAATGCGC ATAAATTATC TCAAGGTTTG
5601   TTTGCATGCT ATCTCTTYTA CTCATATTGC TATCTCGCTC TAACAGTTGC
5651   TATAGTGCTA TCTCACTCGC ACACTATTGC TGTCCTTCAC TAACATCAGG
5701   TCCTGTGTTC CACCTTCAGC TCCAAGGTCT TCGGATCCTC TCCATCATCA
5751   GGTCAGTAGA CCATCAGCTC AGAAGGTCAA AGGTCAGTAG ACCAGAAGGT
5801   CACAGAAGTA GGTCAAGGTC ATATAGAAGG TCAAAGGTCA AGGTCACCGT
5851   GACGTCATCA AGTACTCTAT ACATGCTGAG TACTTGCTTA TACTCTACGA
5901   GTATTGCC(Z)p
```

X et Z représentant les bases A ou C ou G ou T, n représentant un nombre entier pouvant varier de 0 à 50 et p de 0 à 130, cette numérotation ne tenant pas compte des bases X et Z et M représentant les bases A ou C et Y les bases C ou T.

2. Séquence complète de l'ADN viral du Densovirus J-DNV clôné correspondant à la séquence telle que définie à la revendication 1 dans laquelle sont éliminées les deux extrémités "X" et "Z".

3. Plasmides recombinants capables de se répliquer dans Escherichia coli caractérisés en ce qu'ils contiennent une séquence complète ou partielle de l'ADN double ou simple brin selon la revendication 1 ou 2 provoquant une densovirose chez un insecte sensible.

4. Plasmides tels que définis à la revendication 3, caractérisés en ce qu'ils contiennent la séquence complète du génome viral du Densovirus J-DNV, telle que définie à la revendication 2.

5. Plasmide pBRJ tel que défini à la revendication 4 et représenté à la figure 3.

6. Bactérie hôte Escherichia coli caractérisée en ce qu'elle est transformée par un plasmide tel que défini à la revendication 3.

7. Bactérie hôte Escherichia coli, caractérisée en ce qu'elle est transformée par un plasmide tel que défini à la revendication 4.

8. Bactérie hôte Escherichia coli, caractérisée en ce qu'elle est transformée par le plasmide pBRJ tel que défini à la revendication 5.

9. Application à la lutte biologique contre les insectes ravageurs, des plasmides tels que définis à la revendication 3.

10. Application à la lutte biologique contre les insectes ravageurs, des plasmides tels que définis à la revendication 4.

11. Application à la lutte biologique contre les insectes ravageurs du plasmide pBRJ tel que défini à la revendication 5.

**12.** Formulations insecticides contenant les plasmides tels que définis à la revendication 3.

**13.** Formulations insecticides contenant les plasmides tels que définis à la revendication 4.

**14.** Formulations insecticides contenant le plasmide pBRJ tel que défini à la revendication 5.

## Claims

**1.** The complete sequence of the viral genome of the Junonia Densovirus (J-DNV).

1 $(X)_n$GTATTGCCAC CGACGACGAC GACGGCAGTC CCTGCCGGCG AAGCCGGCCG

51 CCGCGAAGCG GCAAGGGACT GCCGTCGTCG TCGTCGGTGG CAATACTCGT

101 AGAGTATAAG CAAGTACTCA GCATGTATAG AGTACTTGAT GACGTCACGG

151 TGACCTTGAC CTTTGACCTT CTATATGACC TTGACCTACT TCTGTGACCT

201 TCTGGTCTAC TGACCTTTGA CCTTCTGAGC TGATGTCTAC TGACCTGATG

251 ATGGAGAGGA TCCGAAGACC TTGGAGCTGA AGGTGGAACA CAGGACCTGA

301 TGTTAGTGAA GGACAGCAAT AGTGTGCGAG TGAGATAGCA CTATAGCAAC

351 TGTTAGAGCG AGATAGCAAT ATGAGTAAAA GAGATAGCAT GCAAACAAAC

401 CTTGAGATAA TTTATGCGCA TTTTATTATC TTGTTATTGT GACCTCGTTT

451 GACCGGCAAA CTCGCGTCGA GGCTGGGCCG TGTGCAAAAC AGGATGTGGC

501 TGGCCAGCGG ACCATTGACT ATATAAAGGC TGACGTGTTC TATTTTTAGT

551 CAGTATGTCT TTCTACACGG CCGGGTTAAT ACATCGTGCG CGACCCGGTT

601 ATCGTATTAT ACCAGAAAGT ACTGCTACTG AAGATATTGA ACTTGGTGCT

651 ATTGGAGAAG AAACTCCATT ATTAAGTGAA GGTGCTGTTA CTGCTGTAGA

701 AGAAAGTGCT GCTGTTGGTT TACCAGAACT TGGTGCTGGA CTTGCTGGTG

751 CTATTGGAAC ACATGCTGAC GTATTGTATA GAAATAGAAA CGTTTTTAAA

801 AGTGTTTTAA CTGGAAATTA TACCGATTTA AAAGGCAATC CTTTAAAACA

851 ACGAAACGCT ATTTCTGAAA AAACTAAACA ACTTGGAAGA GGAATATTTC

901 AAGGCGATTT CAACCGTGCA TTTCCTGATG ATTTAAAATT GGAAACTGAA
951 CAAGAAAAAA AAGATTTACT ACGTTATTAT AATCACAATA GAAGATTAGC
1001 TGGATTAAGT GAAGCTTATC CACAAGGGAA AGGATACGCT TATGCTAAAA
1051 GTCAAAAAGT ATTAGAAGCT GAACGACGAG GATTAACTGT TCCCGGATAT
1101 AAATATCTTG GTCCTGGAAA TTCACTTAAC AGAGGTCAAC CTACTAATCA
1151 AATAGACGAA GACGCTAAGG AACACGACGA AGCATACGAT AAAGCGAAAA
1201 CAAGTCAAGA AGTAAGTCAA GCAGATAATA CATTTGTCAA TAAAGCGTTA
1251 GATCACATAG TTAATGCTAT CAATCTTAAA GAAACTCCTG GTAACGCTTT
1301 TGGAGCTGCT ATCGGAGCTA TTGGAATTGG AACTAAGCAA GCTATCGAAA
1351 AACACAGTGG AGTAATCTAC CCTTCTGTTT CAGGTATGTC CCGTCAAATT
1401 AATTCTAAAT ACTTAAATAG CTGGCATGAC TGGATTGAGC AAAATAAACA
1451 TAATAATTTT GAAGGAATAC AATTACCAGA GGACTTTTAC ACAGAAGAAC
1501 AAACTCTTTC AGATTCACCG ATGTCAGAGG GAACAAAACG TAAAGCTGAT
1551 ACTCCTGTTG AAGAAGGTCC TTCTAAAAAA GGTGCTCATA ACGCTCCACA
1601 TAACTCGCAA GGTACAGATC CTCAAAATCC TAGTTCTTCC GGAGCAACTA
1651 CTTCTTTTGA CGTTGAAATG GCTATGTCAT TACCTGGAAC TGGTTCTGGA
1701 ACATCATCTG GAGGAGGCAA CACTTCAGGT CAAGAGGTTT ATGTAATTCC
1751 TCGTCCATTT TCGAACTTTG GTAAAAAATT AAGTACTTAT ACAAAGTCTC
1801 ATAAATTTAT GATATTTGGT CTTGCCAATA ATGTTATTGG ACCTACAGGT
1851 ACTGGTACAA CAGCTGTAAA TCGTTTAATT ACAACTTGTT TGGCTGAAAT
1901 TCCATGGCAG AAATTGCCTT TGTATATGAA CCAATCTGAA TTTGATTTAT
1951 TACCTCCTGG TAGTAGAGTA GTTGAATGTA ATGTTAAAGT AATATTCAGA
2001 ACTAATCGTA TTGCATTTGA GACTAGTTCA ACTGCTACTA AACAAGCTAC
2051 ATTGAATCAA ATATCTAATT TACAAACTGC TGTTGGATTA AATAAACTTG
2101 GATGGGGTAT TGATAGATCA TTTACTGCTT TTCAATCAGA TCAACCTATG
2151 ATTCCCACTG CTACTAGTGC ACCAAAATAT GAACCTATAA CTGGTACGAC
2201 TGGTTATAGA GGTATGATAG CTGATTATTA TGGTGCTGAT TCTACTAATG
2251 ATGCTGCATT TGGTAATGCT GGTAACTATC CTCATCATCA AGTTGGTTCA
2301 TTTACTTTTA TTCAAAATTA TTATTGTATG TATCAACAAA CCAATCAAGG
2351 TACTGGAGGT TGGCCATGTT TAGCTGAACA TCTTCAACAA TTTGATTCTA

2401 AAACTGTTAA TAATCAATGT TTAATTGATG TAACTTATAA ACCTAAAATG

2451 GGTTTAATTA AACCACCGTT AAATTATAAA ATTATTGGTC AACCTACTGC

2501 AAAAGGTACT ATATCTGTTG GTGATAATTT AGTTAACATG CGAGGAGCTG

2551 TTGTAATAAA TCCACCTGAA GCAACACAAT CTGTTACTGA ATCAACTCAT

2601 AATTTGACTC GCAATTTTCC AGCTAATTTG TTTAATATTT ATTCTGACAT

2651 TGAAAAATCT CAAATTTTAC ATAAAGGACC TTGGGGACAC GAAAATCCAC

2701 AGATACAACC AAGTGTTCAT ATTGGTATTC AAGCTGTACC AGCATTAACT

2751 ACAGGAGCTT TACTTGTAAA TTCAAGTCCT TTAAATTCAT GGACTGATTC

2801 TATGGGTTAT ATTGATGTTA TGTCTAGTTG TACTGTTATG GAATCTCAGC

2851 CTACACACTT TCCATTTTCG ACTGATGCTA ATACTAACCC TGGTAATACC

2901 ATTTATCGTA TTAATCTTAC ACCGAACTCT CTTACTAGTG CTTTCAATGG

2951 ATTGTACGGT AATGGAGCTA CTCTTGGTAA CGTTTAAATA AAACAATAAT

3001 GTATCCCATA ACCATTTATT AAAATGTAAT ATTATATTTA CTCAATAAAA

3051 GGAAAAATGT CATTGGATGT GGTTTCAATT CATAATCCTT TAAGAATGGC

3101 GCAGCATTCC ACTTGTATTG AATAATTCTA TCACTAAAAG CAGTTTCATA

3151 CATAAAAGGT ACAGTATTAT TAGTAAGTAT TATAACTGGA GTGCGTTTTA

3201 CATGTGCATC CATACGATTT TTAACTCTAA CAGTATAAGG ATCTCCTCCA

3251 AACATCATTT TAATTGTATC AGTTAAAGAA CTCTCATAGT TAGGTTCATT

3301 CCATAATAAT ACACGTTTAT TAGGTGCTTC TTGAAATGCA AACAAGTTAT

3351 GTCTATTAGC TTGACCTAAC TGACCATAAG ATAGTAATAA TCCAAAGATC

3401 ATATCAAAAA AGAAATTTTT ACCAGCACTT GGAGGAGATA TAATAAGAAA

3451 AGCATTTAAC TTAGGTATAC GACGGTCTAA TACATTGACC AAATTAGTAA

3501 GAAACTCTAC AATTAAATCT TCATCATCAT TACATTGAAA TTTAAGTAAT

3551 TCTATAATAA TATTCAAAGA ATTCTCAAGA TTATCATATT TCATAGAAGA

3601 AATAAACAAA GCATATGGAT TAAGTTCTTG TTCATCAGTA AAATTATAAT

3651 CTTCAGTAAG TAAATTATAA ATTTCTCTCA AAGACATAGC ATTTAAATCC

3701 TTACCAAAGT CATCGCATGC TGCTTGTATA TAATCACGAT TTTTAGGATC

3751 ACATAACAAA TCATCATCAC GAAATTCTGG CACATCACAT ATAGCACTCA

3801 CTGGAGACAC ATAATATTTT CTTAATAACG CTTTTGTCTT TTTCCGTATG

3851 TATGCGAATT TCCCTGCCGA ATAGGCTTTC TTTTCATAAA GTCTTCCGTT

3901 AGAACTGCCA GCATCTGATC TACGGCTAAT TTTGTGCTCT TGCTGTTCAC

3951 ACTCATAGTA ATCCGTGCAA TCGGAGCTTG ATACCATTTC TCTTTCTTTA

4001 AACTCTCTGG TCCATCGTAC ACATTCATCG TTACTCGGTA TTTTCCCACT

4051 TTCTCCTCTA ACGTATATTG CACGCTCTCC CCGTTTTCGT ACAAAGAAAT

4101 AGATGAAGAC ATCGTACCAG TCTGTTCGCT TGAATTCCCA GATGAATTTG

4151 ACTGGTTTGC CAGTTTTCTG AACAGATCCG AAGGGCTTGA CTTGACTAAT

4201 CCAGATGTCC CTGCAACTGC GATTGGTGTA AGAGCAATCG TGGATGACGT

4251 GGATGTGATC TCCTTCTTCA GAAAATCCGA ACAGTCCGTT TCGTCTACTT

4301 CTTCCGTACT CACGCAAGAC GTCCAAACAT TGATCACGGA GCTGAATATC

4351 TCGTAAGATA ATGACATCGC TGATATATGC GCTGGAAGGT TTAATACTTT

4401 GCCCAGTAAC GTATCCAAAG AATTCACTGC CCATTTTTTC CAGTTCTTCT

4451 GCCATATATT GAAAGTTTTC TTGAGATTCT TTTGCAATGC TGGCCATTGT

4501 TCCTCGTCCT CCATGTGACC ATTCACGTTT TCTAGATGTA TTTGCCACCA

4551 TGCTACATTG TTCACTAGGG GATGTTCTTC CTGAAGACTC TCGTATAATG

4601 TCTTGATTAG GTCTGGTAGT ACTATCGGTT TCTCTGTTGG TGTCTCCATT

4651 GTTCATCTGT AATTAATGTC TACTATTAGG ATGTTTTACA TAAAAACTAT

4701 TAGACATATA TTCGTCCTCT TCTGAACTAC TTGAGTATCT CCTTTTTTTA

4751 TTAGGAGAGT TTTCATAAAT TACAGATATA TTAAACAATG GACAAGTATG

4801 ACATATTTGA CACCAACTAC TACCATCTTG TAATCTATTT ATAATATCTT

4851 CAGCATCTTC TACGAACACA GTTCTATTAT ACATAAATCT GTATTCATCT

4901 GGATCTTCAC AATTAGTATA ACAAAATTTA CAAATTTTCC ACATAGTTTT

4951 AAAGGGCTTT CCATTCATAT TCCATGCATT AAATTCATCA GTATCATAAG

5001 GGTCTCTATG GTTAATTAAT TCTTTAAAAT AATACACACA TTCTATCAGC

5051 ATAGTTTCAT CTAACCATTC AGGTATTTCA TTTAAATGCA TAACAGCTAA

5101 ATATAACTGG AAAGGTAATC TGTTTTGTTT TGTCCAATCC CAGTGTTCTA

5151 ATTCTTCCAT AGCTAAAGTA TGATCTATGT CTTCTCCACA AGCAATAATC

5201 TGATTATCTA GATCATGCTG CATATTAAGT ATAGGTTTAG GCAAAATTGA

5251 CAAGTCTAGA CCATTAAGTC TAGCAGTCTT ATAAGCCTCA TAGAACAAAG

5301 ACTTTGGATT GTACACTTTT TCAAATAAAC GAACGAACAC AAAGAAACCT

5351 GGCAATAGAC ATACCGATTA TATTCTGGAA CCACTTTTGC ACAACACTAC

```
5401    TTTTTCACTG AGATGTTCAC TCGACGACTG CTGCTCGTAG ACTGATGATG
5451    GCGCTCTGCT GTTATCTCTA TTTATAGCCA ATGGTCCGCT GGCCAGCCAC
5501    ATCCTGTTTT GCACACGGCC CAGCCTCGAC GCGAGTTTGC CGGTCAAACG
5551    AGGTCACAAT AACAAGATAA TAAAATGCGC ATAAATTATC TCAAGGTTTG
5601    TTTGCATGCT ATCTCTTYTA CTCATATTGC TATCTCGCTC TAACAGTTGC
5651    TATAGTGCTA TCTCACTCGC ACACTATTGC TGTCCTTCAC TAACATCAGG
5701    TCCTGTGTTC CACCTTCAGC TCCAAGGTCT TCGGATCCTC TCCATCATCA
5751    GGTCAGTAGA CCATCAGCTC AGAAGGTCAA AGGTCAGTAG ACCAGAAGGT
5801    CACAGAAGTA GGTCAAGGTC ATATAGAAGG TCAAAGGTCA AGGTCACCGT
5851    GACGTCATCA AGTACTCTAT ACATGCTGAG TACTTGCTTA TACTCTACGA
5901    GTATTGCC(Z)p
```

X and Z representing the bases A or C or G or T, n representing an integer which can vary from 0 to 50 and p from 0 to 130, this numbering does not take into account bases X and Z and M representing the bases A or C and Y the bases C or T.

**2.** The complete sequence of the viral DNA of the cloned J-DNV Densovirus corresponding to the sequence as defined in claim 1 in which the two "X" and "Z" ends are eliminated.

**3.** Recombinant plasmids capable of replicating on Escherichia coli characterized in that they contain a complete or partial sequence of the double- or single-strand DNA according to claim 1 or 2 causing a densovirus in sensitive insects.

**4.** Plasmids as defined in claim 3, characterized in that they contain the complete sequence of the viral genome of the J-DNV Densovirus, as defined in claim 2.

**5.** Plasmid pBRJ as defined in claim 4 and represented in Figure 3.

**6.** Escherichia coli host bacteria characterized in that it is transformed by a plasmid as defined in claim 3.

**7.** Escherichia coli host bacteria characterized in that it is transformed by a plasmid as defined in claim 4.

**8.** Escherichia coli host bacteria characterized in that it is transformed by the plasmid pBRJ as defined in claim 5.

**9.** Use of plasmids as defined in claim 3 in the biological combating of insects which devastate crops.

**10.** Use of plasmids as defined in claim 4 in the biological combating of insects which devastate crops.

**11.** Use of plasmid pBRJ as defined in claim 5 in the biological combating of insects which devastate crops.

**12.** Insecticide formulations containing the plasmids as defined in claim 3.

**13.** Insecticide formulations containing the plasmids as defined in claim 4.

**14.** Insecticide formulations containing the plasmid pBRJ as defined in claim 5.

**Patentansprüche**

**1.** Vollständige Sequenz des viralen Genoms des Junonia-Densovirus (J-DNV)

```
  1   (X)nGTATTGCCAC CGACGACGAC GACGGCAGTC CCTGCCGGCG AAGCCGGCCG
 51       CCGCGAAGCG GCAAGGGACT GCCGTCGTCG TCGTCGGTGG CAATACTCGT
101       AGAGTATAAG CAAGTACTCA GCATGTATAG AGTACTTGAT GACGTCACGG
151       TGACCTTGAC CTTTGACCTT CTATATGACC TTGACCTACT TCTGTGACCT
201       TCTGGTCTAC TGACCTTTGA CCTTCTGAGC TGATGTCTAC TGACCTGATG
251       ATGGAGAGGA TCCGAAGACC TTGGAGCTGA AGGTGGAACA CAGGACCTGA
301       TGTTAGTGAA GGACAGCAAT AGTGTGCGAG TGAGATAGCA CTATAGCAAC
351       TGTTAGAGCG AGATAGCAAT ATGAGTAAAA GAGATAGCAT GCAAACAAAC
401       CTTGAGATAA TTTATGCGCA TTTTATTATC TTGTTATTGT GACCTCGTTT
451       GACCGGCAAA CTCGCGTCGA GGCTGGGCCG TGTGCAAAAC AGGATGTGGC
501       TGGCCAGCGG ACCATTGACT ATATAAAGGC TGACGTGTTC TATTTTTAGT
551       CAGTATGTCT TTCTACACGG CCGGGTTAAT ACATCGTGCG CGACCCGGTT
601       ATCGTATTAT ACCAGAAAGT ACTGCTACTG AAGATATTGA ACTTGGTGCT
651       ATTGGAGAAG AAACTCCATT ATTAAGTGAA GGTGCTGTTA CTGCTGTAGA
701       AGAAAGTGCT GCTGTTGGTT TACCAGAACT TGGTGCTGGA CTTGCTGGTG
751       CTATTGGAAC ACATGCTGAC GTATTGTATA GAAATAGAAA CGTTTTTAAA
801       AGTGTTTTAA CTGGAAATTA TACCGATTTA AAAGGCAATC CTTTAAAACA
851       ACGAAACGCT ATTTCTGAAA AAACTAAACA ACTTGGAAGA GGAATATTTC
```

901 AAGGCGATTT CAACCGTGCA TTTCCTGATG ATTTAAAATT GGAAACTGAA
951 CAAGAAAAAA AAGATTTACT ACGTTATTAT AATCACAATA GAAGATTAGC
1001 TGGATTAAGT GAAGCTTATC CACAAGGGAA AGGATACGCT TATGCTAAAA
1051 GTCAAAAAGT ATTAGAAGCT GAACGACGAG GATTAACTGT TCCCGGATAT
1101 AAATATCTTG GTCCTGGAAA TTCACTTAAC AGAGGTCAAC CTACTAATCA
1151 AATAGACGAA GACGCTAAGG AACACGACGA AGCATACGAT AAAGCGAAAA
1201 CAAGTCAAGA AGTAAGTCAA GCAGATAATA CATTTGTCAA TAAAGCGTTA
1251 GATCACATAG TTAATGCTAT CAATCTTAAA GAAACTCCTG GTAACGCTTT
1301 TGGAGCTGCT ATCGGAGCTA TTGGAATTGG AACTAAGCAA GCTATCGAAA
1351 AACACAGTGG AGTAATCTAC CCTTCTGTTT CAGGTATGTC CCGTCAAATT
1401 AATTCTAAAT ACTTAAATAG CTGGCATGAC TGGATTGAGC AAAATAAACA
1451 TAATAATTTT GAAGGAATAC AATTACCAGA GGACTTTTAC ACAGAAGAAC
1501 AAACTCTTTC AGATTCACCG ATGTCAGAGG GAACAAAACG TAAAGCTGAT
1551 ACTCCTGTTG AAGAAGGTCC TTCTAAAAAA GGTGCTCATA ACGCTCCACA
1601 TAACTCGCAA GGTACAGATC CTCAAAATCC TAGTTCTTCC GGAGCAACTA
1651 CTTCTTTTGA CGTTGAAATG GCTATGTCAT TACCTGGAAC TGGTTCTGGA
1701 ACATCATCTG GAGGAGGCAA CACTTCAGGT CAAGAGGTTT ATGTAATTCC
1751 TCGTCCATTT TCGAACTTTG GTAAAAAATT AAGTACTTAT ACAAAGTCTC
1801 ATAAATTTAT GATATTTGGT CTTGCCAATA ATGTTATTGG ACCTACAGGT
1851 ACTGGTACAA CAGCTGTAAA TCGTTTAATT ACAACTTGTT TGGCTGAAAT
1901 TCCATGGCAG AAATTGCCTT TGTATATGAA CCAATCTGAA TTTGATTTAT
1951 TACCTCCTGG TAGTAGAGTA GTTGAATGTA ATGTTAAAGT AATATTCAGA
2001 ACTAATCGTA TTGCATTTGA GACTAGTTCA ACTGCTACTA AACAAGCTAC
2051 ATTGAATCAA ATATCTAATT TACAAACTGC TGTTGGATTA AATAAACTTG
2101 GATGGGGTAT TGATAGATCA TTTACTGCTT TTCAATCAGA TCAACCTATG
2151 ATTCCCACTG CTACTAGTGC ACCAAAATAT GAACCTATAA CTGGTACGAC
2201 TGGTTATAGA GGTATGATAG CTGATTATTA TGGTGCTGAT TCTACTAATG
2251 ATGCTGCATT TGGTAATGCT GGTAACTATC CTCATCATCA AGTTGGTTCA
2301 TTTACTTTTA TTCAAAATTA TTATTGTATG TATCAACAAA CCAATCAAGG
2351 TACTGGAGGT TGGCCATGTT TAGCTGAACA TCTTCAACAA TTTGATTCTA

2401 AAACTGTTAA TAATCAATGT TTAATTGATG TAACTTATAA ACCTAAAATG
2451 GGTTTAATTA AACCACCGTT AAATTATAAA ATTATTGGTC AACCTACTGC
2501 AAAAGGTACT ATATCTGTTG GTGATAATTT AGTTAACATG CGAGGAGCTG
2551 TTGTAATAAA TCCACCTGAA GCAACACAAT CTGTTACTGA ATCAACTCAT
2601 AATTTGACTC GCAATTTTCC AGCTAATTTG TTTAATATTT ATTCTGACAT
2651 TGAAAAATCT CAAATTTTAC ATAAAGGACC TTGGGGACAC GAAAATCCAC
2701 AGATACAACC AAGTGTTCAT ATTGGTATTC AAGCTGTACC AGCATTAACT
2751 ACAGGAGCTT TACTTGTAAA TTCAAGTCCT TTAAATTCAT GGACTGATTC
2801 TATGGGTTAT ATTGATGTTA TGTCTAGTTG TACTGTTATG GAATCTCAGC
2851 CTACACACTT TCCATTTTCG ACTGATGCTA ATACTAACCC TGGTAATACC
2901 ATTTATCGTA TTAATCTTAC ACCGAACTCT CTTACTAGTG CTTTCAATGG
2951 ATTGTACGGT AATGGAGCTA CTCTTGGTAA CGTTTAAATA AAACAATAAT
3001 GTATCCCATA ACCATTTATT AAAATGTAAT ATTATATTTA CTCAATAAAA
3051 GGAAAAATGT CATTGGATGT GGTTTCAATT CATAATCCTT TAAGAATGGC
3101 GCAGCATTCC ACTTGTATTG AATAATTCTA TCACTAAAAG CAGTTTCATA
3151 CATAAAAGGT ACAGTATTAT TAGTAAGTAT TATAACTGGA GTGCGTTTTA
3201 CATGTGCATC CATACGATTT TTAACTCTAA CAGTATAAGG ATCTCCTCCA
3251 AACATCATTT TAATTGTATC AGTTAAAGAA CTCTCATAGT TAGGTTCATT
3301 CCATAATAAT ACACGTTTAT TAGGTGCTTC TTGAAATGCA AACAAGTTAT
3351 GTCTATTAGC TTGACCTAAC TGACCATAAG ATAGTAATAA TCCAAAGATC
3401 ATATCAAAAA AGAAATTTTT ACCAGCACTT GGAGGAGATA TAATAAGAAA
3451 AGCATTTAAC TTAGGTATAC GACGGTCTAA TACATTGACC AAATTAGTAA
3501 GAAACTCTAC AATTAAATCT TCATCATCAT TACATTGAAA TTTAAGTAAT
3551 TCTATAATAA TATTCAAAGA ATTCTCAAGA TTATCATATT TCATAGAAGA
3601 AATAAACAAA GCATATGGAT TAAGTTCTTG TTCATCAGTA AAATTATAAT
3651 CTTCAGTAAG TAAATTATAA ATTTCTCTCA AAGACATAGC ATTTAAATCC
3701 TTACCAAAGT CATCGCATGC TGCTTGTATA TAATCACGAT TTTTAGGATC
3751 ACATAACAAA TCATCATCAC GAAATTCTGG CACATCACAT ATAGCACTCA
3801 CTGGAGACAC ATAATATTTT CTTAATAACG CTTTTGTCTT TTTCCGTATG
3851 TATGCGAATT TCCCTGCCGA ATAGGCTTTC TTTTCATAAA GTCTTCCGTT

```
3901   AGAACTGCCA GCATCTGATC TACGGCTAAT TTTGTGCTCT TGCTGTTCAC
3951   ACTCATAGTA ATCCGTGCAA TCGGAGCTTG ATACCATTTC TCTTTCTTTA
4001   AACTCTCTGG TCCATCGTAC ACATTCATCG TTACTCGGTA TTTTCCCACT
4051   TTCTCCTCTA ACGTATATTG CACGCTCTCC CCGTTTTCGT ACAAAGAAAT
4101   AGATGAAGAC ATCGTACCAG TCTGTTCGCT TGAATTCCCA GATGAATTTG
4151   ACTGGTTTGC CAGTTTTCTG AACAGATCCG AAGGGCTTGA CTTGACTAAT
4201   CCAGATGTCC CTGCAACTGC GATTGGTGTA AGAGCAATCG TGGATGACGT
4251   GGATGTGATC TCCTTCTTCA GAAAATCCGA ACAGTCCGTT TCGTCTACTT
4301   CTTCCGTACT CACGCAAGAC GTCCAAACAT TGATCACGGA GCTGAATATC
4351   TCGTAAGATA ATGACATCGC TGATATATGC GCTGGAAGGT TTAATACTTT
4401   GCCCAGTAAC GTATCCAAAG AATTCACTGC CCATTTTTTC CAGTTCTTCT
4451   GCCATATATT GAAAGTTTTC TTGAGATTCT TTTGCAATGC TGGCCATTGT
4501   TCCTCGTCCT CCATGTGACC ATTCACGTTT TCTAGATGTA TTTGCCACCA
4551   TGCTACATTG TTCACTAGGG GATGTTCTTC CTGAAGACTC TCGTATAATG
4601   TCTTGATTAG GTCTGGTAGT ACTATCGGTT TCTCTGTTGG TGTCTCCATT
4651   GTTCATCTGT AATTAATGTC TACTATTAGG ATGTTTTACA TAAAAACTAT
4701   TAGACATATA TTCGTCCTCT TCTGAACTAC TTGAGTATCT CCTTTTTTTA
4751   TTAGGAGAGT TTTCATAAAT TACAGATATA TTAAACAATG GACAAGTATG
4801   ACATATTTGA CACCAACTAC TACCATCTTG TAATCTATTT ATAATATCTT
4851   CAGCATCTTC TACGAACACA GTTCTATTAT ACATAAATCT GTATTCATCT
4901   GGATCTTCAC AATTAGTATA ACAAAATTTA CAAATTTTCC ACATAGTTTT
4951   AAAGGGCTTT CCATTCATAT TCCATGCATT AAATTCATCA GTATCATAAG
5001   GGTCTCTATG GTTAATTAAT TCTTTAAAAT AATACACACA TTCTATCAGC
5051   ATAGTTTCAT CTAACCATTC AGGTATTTCA TTTAAATGCA TAACAGCTAA
5101   ATATAACTGG AAAGGTAATC TGTTTTGTTT TGTCCAATCC CAGTGTTCTA
5151   ATTCTTCCAT AGCTAAAGTA TGATCTATGT CTTCTCCACA AGCAATAATC
5201   TGATTATCTA GATCATGCTG CATATTAAGT ATAGGTTTAG GCAAAATTGA
5251   CAAGTCTAGA CCATTAAGTC TAGCAGTCTT ATAAGCCTCA TAGAACAAAG
5301   ACTTTGGATT GTACACTTTT TCAAATAAAC GAACGAACAC AAAGAAACCT
5351   GGCAATAGAC ATACCGATTA TATTCTGGAA CCACTTTTGC ACAACACTAC
```

```
5401   TTTTTCACTG AGATGTTCAC TCGACGACTG CTGCTCGTAG ACTGATGATG
5451   GCGCTCTGCT GTTATCTCTA TTTATAGCCA ATGGTCCGCT GGCCAGCCAC
5501   ATCCTGTTTT GCACACGGCC CAGCCTCGAC GCGAGTTTGC CGGTCAAACG
5551   AGGTCACAAT AACAAGATAA TAAAATGCGC ATAAATTATC TCAAGGTTTG
5601   TTTGCATGCT ATCTCTTTTA CTCATATTGC TATCTCGCTC TAACAGTTGC
5651   TATAGTGCTA TCTCACTCGC ACACTATTGC TGTCCTTCAC TAACATCAGG
5701   TCCTGTGTTC CACCTTCAGC TCCAAGGTCT TCGGATCCTC TCCATCATCA
5751   GGTCAGTAGA CCATCAGCTC AGAAGGTCAA AGGTCAGTAG ACCAGAAGGT
5801   CACAGAAGTA GGTCAAGGTC ATATAGAAGG TCAAAGGTCA AGGTCACCGT
5851   GACGTCATCA AGTACTCTAT ACATGCTGAG TACTTGCTTA TACTCTACGA
5901   GTATTGCC(Z)p
```

worin X und Z für die Basen A, C, G oder T stehen, n für eine ganze Zahl zwischen 0 und 50, und p zwischen 0 und 130 stehen, wobei diese Zählung die Basen X und Z nicht berücksichtigt, und M für die Basen A oder C steht, und Y für die Basen C oder T steht.

**2.** Vollständige Sequenz der viralen DNA des Densovirus J-DNV, cloniert entsprechend der Sequenz nach Anspruch 1, worin die zwei Enden "X" und "Z" entfernt sind.

**3.** Recombinante Plasmide mit der Fähigkeit zur Replikation in Escherichia coli, **dadurch gekennzeichnet**, daß sie eine vollständige oder teilweise, doppel- oder einzelsträngige DNA-Sequenz nach Anspruch 1 oder 2 enthalten, welche bei einem empfindlichen Insekt eine Densovirose hervorrufen.

**4.** Plasmide nach Anspruch 3, dadurch gekennzeichnet, daß sie die vollständige Sequenz des viralen Genoms des Densovirus J-DNV nach Anspruch 2 enthalten.

**5.** Plasmid pBRJ nach Anspruch 4 und dargestellt in Figur 3.

**6.** Bakterieller Wirt Escherichia coli, **dadurch gekennzeichnet**, daß er durch ein Plasmid nach Anspruch 3 transformiert wurde.

**7.** Bakterieller Wirt Escherichia coli, **dadurch gekennzeichnet**, daß er durch ein Plasmid nach Anspruch 4 transformiert wurde.

**8.** Bakterieller Wirt Escherichia coli, **dadurch gekennzeichnet**, daß er durch das Plasmid pBRJ nach Anspruch 5 transformiert wurde.

**9.** Verwendung der Plasmide nach Anspruch 3 zur biologischen Bekämpfung von schädigenden Insekten.

**10.** Verwendung der Plasmide nach Anspruch 4 zur biologischen Bekämpfung von schädigenden Insekten.

**11.** Verwendung des Plasmids pBRJ nach Anspruch 5 zur biologischen Bekämpfung von schädigenden Insekten.

**12.** Insektizide Formulierungen, enthaltend die Plasmide nach Anspruch 3.

**13.** Insektizide Formulierungen, enthaltend die Plasmide nach Anspruch 4.

**14.** Insektizide Formulierungen, enthaltend das Plasmid pBRJ nach Anspruch 5.

1 $(X)_n$GTATTGCCAC CGACGACGAC GACGGCAGTC CCTGCCGGCG AAGCCGGCCG

51 CCGCGAAGCG GCAAGGGACT GCCGTCGTCG TCGTCGGTGG CAATACTCGT

101 AGAGTATAAG CAAGTACTCA GCATGTATAG AGTACTTGAT GACGTCACGG

151 TGACCTTGAC CTTTGACCTT CTATATGACC TTGACCTACT TCTGTGACCT

201 TCTGGTCTAC TGACCTTTGA CCTTCTGAGC TGATGTCTAC TGACCTGATG

251 ATGGAGAGGA TCCGAAGACC TTGGAGCTGA AGGTGGAACA CAGGACCTGA

301 TGTTAGTGAA GGACAGCAAT AGTGTGCGAG TGAGATAGCA CTATAGCAAC

351 TGTTAGAGCG AGATAGCAAT ATGAGTAAAA GAGATAGCAT GCAAACAAAC

401 CTTGAGATAA TTTATGCGCA TTTTATTATC TTGTTATTGT GACCTCGTTT

451 GACCGGCAAA CTCGCGTCGA GGCTGGGCCG TGTGCAAAAC AGGATGTGGC

501 TGGCCAGCGG ACCATTGACT ATATAAAGGC TGACGTGTTC TATTTTTAGT

551 CAGTATGTCT TTCTACACGG CCGGGTTAAT ACATCGTGCG CGACCCGGTT

601 ATCGTATTAT ACCAGAAAGT ACTGCTACTG AAGATATTGA ACTTGGTGCT

651 ATTGGAGAAG AAACTCCATT ATTAAGTGAA GGTGCTGTTA CTGCTGTAGA

701 AGAAAGTGCT GCTGTTGGTT TACCAGAACT TGGTGCTGGA CTTGCTGGTG

751 CTATTGGAAC ACATGCTGAC GTATTGTATA GAAATAGAAA CGTTTTTAAA

801 AGTGTTTTAA CTGGAAATTA TACCGATTTA AAAGGCAATC CTTTAAAACA

851 ACGAAACGCT ATTTCTGAAA AAACTAAACA ACTTGGAAGA GGAATATTTC

901 AAGGCGATTT CAACCGTGCA TTTCCTGATG ATTTAAAATT GGAAACTGAA

951 CAAGAAAAAA AAGATTTACT ACGTTATTAT AATCACAATA GAAGATTAGC

1001 TGGATTAAGT GAAGCTTATC CACAAGGGAA AGGATACGCT TATGCTAAAA

1051 GTCAAAAAGT ATTAGAAGCT GAACGACGAG GATTAACTGT TCCCGGATAT

1101 AAATATCTTG GTCCTGGAAA TTCACTTAAC AGAGGTCAAC CTACTAATCA

1151 AATAGACGAA GACGCTAAGG AACACGACGA AGCATACGAT AAAGCGAAAA

1201 CAAGTCAAGA AGTAAGTCAA GCAGATAATA CATTTGTCAA TAAAGCGTTA

1251 GATCACATAG TTAATGCTAT CAATCTTAAA GAAACTCCTG GTAACGCTTT

1301 TGGAGCTGCT ATCGGAGCTA TTGGAATTGG AACTAAGCAA GCTATCGAAA

1351 AACACAGTGG AGTAATCTAC CCTTCTGTTT CAGGTATGTC CCGTCAAATT

1401 AATTCTAAAT ACTTAAATAG CTGGCATGAC TGGATTGAGC AAAATAAACA

1451 TAATAATTTT GAAGGAATAC AATTACCAGA GGACTTTTAC ACAGAAGAAC

1501 AAACTCTTTC AGATTCACCG ATGTCAGAGG GAACAAAACG TAAAGCTGAT

1551 ACTCCTGTTG AAGAAGGTCC TTCTAAAAAA GGTGCTCATA ACGCTCCACA

FIGURE 1

1601 TAACTCGCAA GGTACAGATC CTCAAAATCC TAGTTCTTCC GGAGCAACTA
1651 CTTCTTTTGA CGTTGAAATG GCTATGTCAT TACCTGGAAC TGGTTCTGGA
1701 ACATCATCTG GAGGAGGCAA CACTTCAGGT CAAGAGGTTT ATGTAATTCC
1751 TCGTCCATTT TCGAACTTTG GTAAAAAATT AAGTACTTAT ACAAAGTCTC
1801 ATAAATTTAT GATATTTGGT CTTGCCAATA ATGTTATTGG ACCTACAGGT
1851 ACTGGTACAA CAGCTGTAAA TCGTTTAATT ACAACTTGTT TGGCTGAAAT
1901 TCCATGGCAG AAATTGCCTT TGTATATGAA CCAATCTGAA TTTGATTTAT
1951 TACCTCCTGG TAGTAGAGTA GTTGAATGTA ATGTTAAAGT AATATTCAGA
2001 ACTAATCGTA TTGCATTTGA GACTAGTTCA ACTGCTACTA AACAAGCTAC
2051 ATTGAATCAA ATATCTAATT TACAAACTGC TGTTGGATTA AATAAACTTG
2101 GATGGGGTAT TGATAGATCA TTTACTGCTT TTCAATCAGA TCAACCTATG
2151 ATTCCCACTG CTACTAGTGC ACCAAAATAT GAACCTATAA CTGGTACGAC
2201 TGGTTATAGA GGTATGATAG CTGATTATTA TGGTGCTGAT TCTACTAATG
2251 ATGCTGCATT TGGTAATGCT GGTAACTATC CTCATCATCA AGTTGGTTCA
2301 TTTACTTTTA TTCAAAATTA TTATTGTATG TATCAACAAA CCAATCAAGG
2351 TACTGGAGGT TGGCCATGTT TAGCTGAACA TCTTCAACAA TTTGATTCTA
2401 AAACTGTTAA TAATCAATGT TTAATTGATG TAACTTATAA ACCTAAAATG
2451 GGTTTAATTA AACCACCGTT AAATTATAAA ATTATTGGTC AACCTACTGC
2501 AAAAGGTACT ATATCTGTTG GTGATAATTT AGTTAACATG CGAGGAGCTG
2551 TTGTAATAAA TCCACCTGAA GCAACACAAT CTGTTACTGA ATCAACTCAT
2601 AATTTGACTC GCAATTTTCC AGCTAATTTG TTTAATATTT ATTCTGACAT
2651 TGAAAAATCT CAAATTTTAC ATAAAGGACC TTGGGGACAC GAAAATCCAC
2701 AGATACAACC AAGTGTTCAT ATTGGTATTC AAGCTGTACC AGCATTAACT
2751 ACAGGAGCTT TACTTGTAAA TTCAAGTCCT TTAAATTCAT GGACTGATTC
2801 TATGGGTTAT ATTGATGTTA TGTCTAGTTG TACTGTTATG GAATCTCAGC
2851 CTACACACTT TCCATTTTCG ACTGATGCTA ATACTAACCC TGGTAATACC
2901 ATTTATCGTA TTAATCTTAC ACCGAACTCT CTTACTAGTG CTTTCAATGG
2951 ATTGTACGGT AATGGAGCTA CTCTTGGTAA CGTTTAAATA AAACAATAAT
3001 GTATCCCATA ACCATTTATT AAAATGTAAT ATTATATTTA CTCAATAAAA
3051 GGAAAAATGT CATTGGATGT GGTTTCAATT CATAATCCTT TAAGAATGGC
3101 GCAGCATTCC ACTTGTATTG AATAATTCTA TCACTAAAAG CAGTTTCATA
3151 CATAAAAGGT ACAGTATTAT TAGTAAGTAT TATAACTGGA GTGCGTTTTA

FIGURE 1

3201 CATGTGCATC CATACGATTT TTAACTCTAA CAGTATAAGG ATCTCCTCCA
3251 AACATCATTT TAATTGTATC AGTTAAAGAA CTCTCATAGT TAGGTTCATT
3301 CCATAATAAT ACACGTTTAT TAGGTGCTTC TTGAAATGCA AACAAGTTAT
3351 GTCTATTAGC TTGACCTAAC TGACCATAAG ATAGTAATAA TCCAAAGATC
3401 ATATCAAAAA AGAAATTTTT ACCAGCACTT GGAGGAGATA TAATAAGAAA
3451 AGCATTTAAC TTAGGTATAC GACGGTCTAA TACATTGACC AAATTAGTAA
3501 GAAACTCTAC AATTAAATCT TCATCATCAT TACATTGAAA TTTAAGTAAT
3551 TCTATAATAA TATTCAAAGA ATTCTCAAGA TTATCATATT TCATAGAAGA
3601 AATAAACAAA GCATATGGAT TAAGTTCTTG TTCATCAGTA AAATTATAAT
3651 CTTCAGTAAG TAAATTATAA ATTTCTCTCA AAGACATAGC ATTTAAATCC
3701 TTACCAAAGT CATCGCATGC TGCTTGTATA TAATCACGAT TTTTAGGATC
3751 ACATAACAAA TCATCATCAC GAAATTCTGG CACATCACAT ATAGCACTCA
3801 CTGGAGACAC ATAATATTTT CTTAATAACG CTTTTGTCTT TTTCCGTATG
3851 TATGCGAATT TCCCTGCCGA ATAGGCTTTC TTTTCATAAA GTCTTCCGTT
3901 AGAACTGCCA GCATCTGATC TACGGCTAAT TTTGTGCTCT TGCTGTTCAC
3951 ACTCATAGTA ATCCGTGCAA TCGGAGCTTG ATACCATTTC TCTTTCTTTA
4001 AACTCTCTGG TCCATCGTAC ACATTCATCG TTACTCGGTA TTTTCCCACT
4051 TTCTCCTCTA ACGTATATTG CACGCTCTCC CCGTTTTCGT ACAAAGAAAT
4101 AGATGAAGAC ATCGTACCAG TCTGTTCGCT TGAATTCCCA GATGAATTTG
4151 ACTGGTTTGC CAGTTTTCTG AACAGATCCG AAGGGCTTGA CTTGACTAAT
4201 CCAGATGTCC CTGCAACTGC GATTGGTGTA AGAGCAATCG TGGATGACGT
4251 GGATGTGATC TCCTTCTTCA GAAAATCCGA ACAGTCCGTT TCGTCTACTT
4301 CTTCCGTACT CACGCAAGAC GTCCAAACAT TGATCACGGA GCTGAATATC
4351 TCGTAAGATA ATGACATCGC TGATATATGC GCTGGAAGGT TTAATACTTT
4401 GCCCAGTAAC GTATCCAAAG AATTCACTGC CCATTTTTTC CAGTTCTTCT
4451 GCCATATATT GAAAGTTTTC TTGAGATTCT TTTGCAATGC TGGCCATTGT
4501 TCCTCGTCCT CCATGTGACC ATTCACGTTT TCTAGATGTA TTTGCCACCA
4551 TGCTACATTG TTCACTAGGG GATGTTCTTC CTGAAGACTC TCGTATAATG
4601 TCTTGATTAG GTCTGGTAGT ACTATCGGTT TCTCTGTTGG TGTCTCCATT
4651 GTTCATCTGT AATTAATGTC TACTATTAGG ATGTTTTACA TAAAAACTAT
4701 TAGACATATA TTCGTCCTCT TCTGAACTAC TTGAGTATCT CCTTTTTTTA
4751 TTAGGAGAGT TTTCATAAAT TACAGATATA TTAAACAATG GACAAGTATG

FIGURE 1

| | |
|---|---|
| 4801 | ACATATTTGA CACCAACTAC TACCATCTTG TAATCTATTT ATAATATCTT |
| 4851 | CAGCATCTTC TACGAACACA GTTCTATTAT ACATAAATCT GTATTCATCT |
| 4901 | GGATCTTCAC AATTAGTATA ACAAAATTTA CAAATTTTCC ACATAGTTTT |
| 4951 | AAAGGGCTTT CCATTCATAT TCCATGCATT AAATTCATCA GTATCATAAG |
| 5001 | GGTCTCTATG GTTAATTAAT TCTTTAAAAT AATACACACA TTCTATCAGC |
| 5051 | ATAGTTTCAT CTAACCATTC AGGTATTTCA TTTAAATGCA TAACAGCTAA |
| 5101 | ATATAACTGG AAAGGTAATC TGTTTTGTTT TGTCCAATCC CAGTGTTCTA |
| 5151 | ATTCTTCCAT AGCTAAAGTA TGATCTATGT CTTCTCCACA AGCAATAATC |
| 5201 | TGATTATCTA GATCATGCTG CATATTAAGT ATAGGTTTAG GCAAAATTGA |
| 5251 | CAAGTCTAGA CCATTAAGTC TAGCAGTCTT ATAAGCCTCA TAGAACAAAG |
| 5301 | ACTTTGGATT GTACACTTTT TCAAATAAAC GAACGAACAC AAAGAAACCT |
| 5351 | GGCAATAGAC ATACCGATTA TATTCTGGAA CCACTTTTGC ACAACACTAC |
| 5401 | TTTTTCACTG AGATGTTCAC TCGACGACTG CTGCTCGTAG ACTGATGATG |
| 5451 | GCGCTCTGCT GTTATCTCTA TTTATAGCCA ATGGTCCGCT GGCCAGCCAC |
| 5501 | ATCCTGTTTT GCACACGGCC CAGCCTCGAC GCGAGTTTGC CGGTCAAACG |
| 5551 | AGGTCACAAT AACAAGATAA TAAAATGCGC ATAAATTATC TCAAGGTTTG |
| 5601 | TTTGCATGCT ATCTCTTYTA CTCATATTGC TATCTCGCTC TAACAGTTGC |
| 5651 | TATAGTGCTA TCTCACTCGC ACACTATTGC TGTCCTTCAC TAACATCAGG |
| 5701 | TCCTGTGTTC CACCTTCAGC TCCAAGGTCT TCGGATCCTC TCCATCATCA |
| 5751 | GGTCAGTAGA CCATCAGCTC AGAAGGTCAA AGGTCAGTAG ACCAGAAGGT |
| 5801 | CACAGAAGTA GGTCAAGGTC ATATAGAAGG TCAAAGGTCA AGGTCACCGT |
| 5851 | GACGTCATCA AGTACTCTAT ACATGCTGAG TACTTGCTTA TACTCTACGA |
| 5901 | GTATTGCC$(Z)_p$ |

FIGURE 1

La figure 1 représente la séquence nucléotidique complète du génome viral du Densovirus J-DNV. X et Z représentent les bases A ou C ou G ou T, n est un nombre entier compris entre 0 et 50 et p est un nombre entier compris entre 0 et 130. La numérotation ne tient pas compte des bases X et Z. M représente A ou C et Y, C ou T.

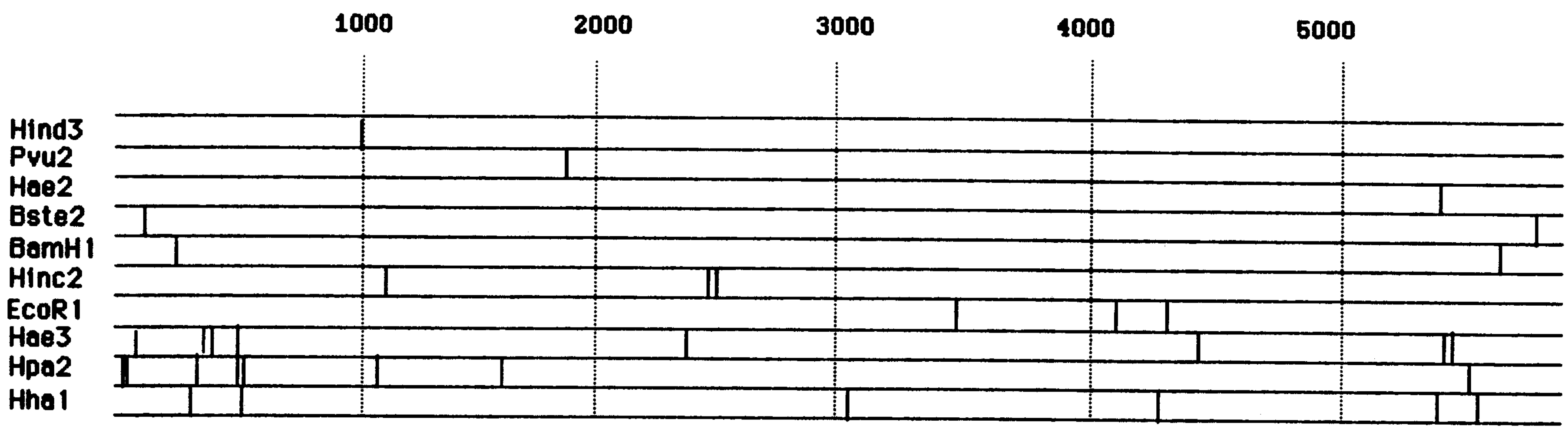


Les enzymes de restriction Apa1, Bgl2, Cla1, Kpn1, Pst1, Pvu1, Sal1 et Sma1 n'ont pas de site de coupure dans le génome du Densovirus de Junonia

Figure2: Carte de restriction du Génome du Densovirus de Junonia avec les enzymes de restriction Hind3, Pvu2, Hae2, Bste2, BamH, Hinc2, EcoR1, Hae3, Hpa2 Hha1, Apa1, Bgl2, Cla1, Kpn1, Pst1, Pvu1, Sal1, Sma1

FIGURE 2

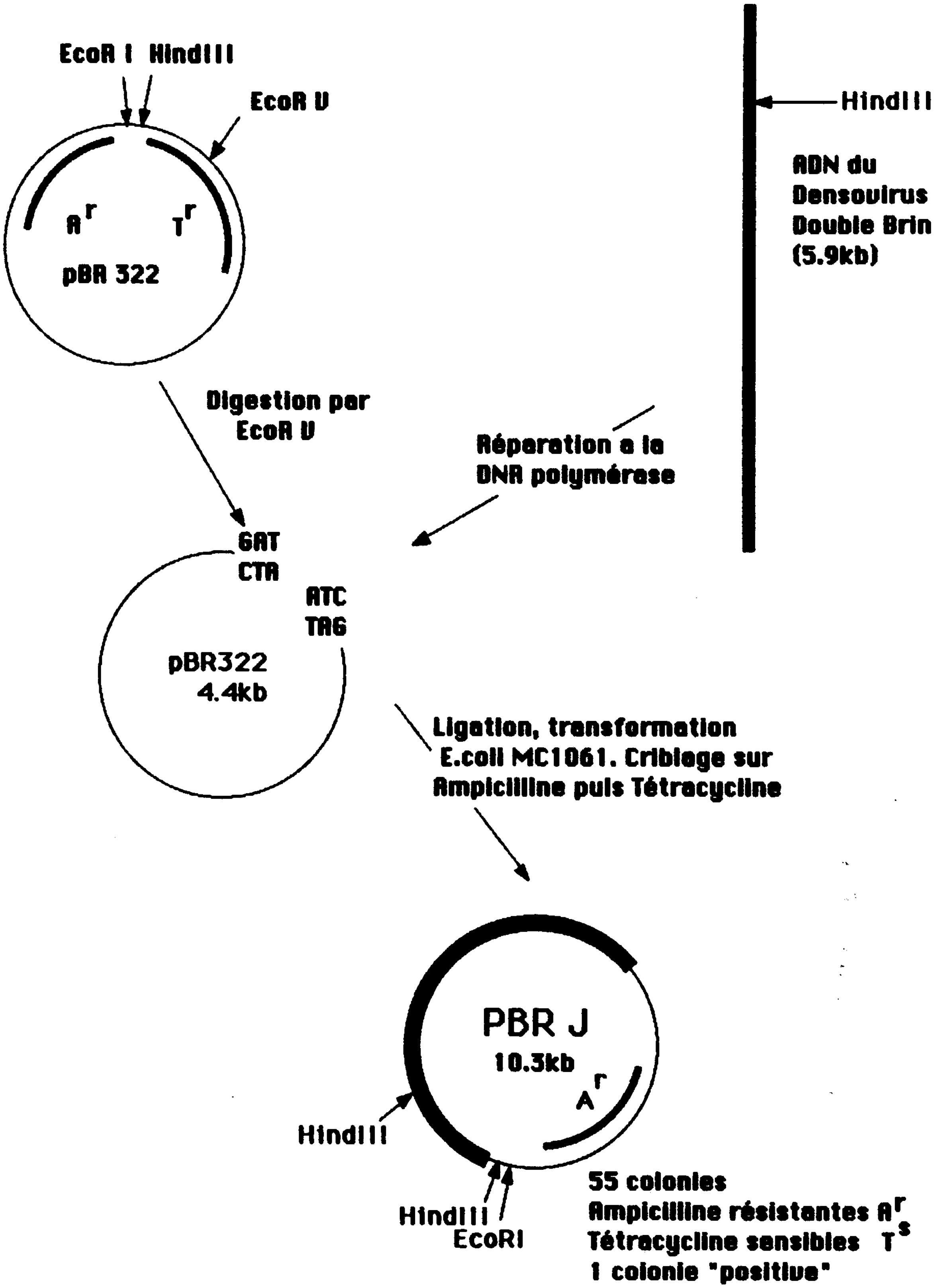


Figure 3: Schéma de la construction de pBRJ